# EUROPEAN PATENT SPECIFICATION

(11) **EP 3 146 059 B1**
(45) Date of publication and mention of the grant of the patent: **18.09.2019**
(21) Application number: 15795634.3
(22) Date of filing: 22.05.2015
(51) Int. Cl.: C12N 1/00, C12N 15/01, C12P 7/64, C12R 1/90, A23D 9/00, C10G 3/00, C11B 1/00

(54) **LABYRINTHULOMYCETE STRAINS FOR PRODUCING DOCOSAHEXAENOIC ACID AND MYRISTIC ACID**
LABYRINTHULOMYCET-STÄMME ZUR HERSTELLUNG VON DECOSAHEXAENSÄURE UND MYRISTINSÄURE
SOUCHES DE LABYRINTHULOMYCETES POUR LA PRODUCTION D'ACIDE DOCOSAHEXAÉNOÏQUE ET D'ACIDE MYRISTIQUE

(30) Priority: 22.05.2014 US 201462002107 P
(43) Date of publication of application: 29.03.2017
(73) Proprietor: Synthetic Genomics, Inc., La Jolla, CA 92037 (US)
(72) Inventor: RADAKOVITS, Randor, R., Escondido, CA 92026 (US); CHAMPAGNE, Michele, M., San Diego, CA 92024 (US)
(74) Representative: Newton, Michael Jonathan
(86) International application number: PCT/US2015/032308
(87) International publication number: WO 2015/179844

(56) References cited:
- WO-A2-2007/068997
- US-A1- 2010 285 105
- US-A1- 2013 344 546
- CHANG ET AL: "Comparison of Thraustochytrids Aurantiochytrium sp., Schizochytrium sp., Thraustochytrium sp., and Ulkenia sp. for production of biodiesel, long-chain omega-3 oils, and exopolysaccharide", MARINE BIOTECHNOLOGY, vol. 16, 25 January 2014 (2014-01-25), pages 396-411, XP002761877,
- RADAKOVITS ET AL: "Genetic engineering of fatty acid chain length in Phaeodactylum tricornutum", METABOLIC ENGINEERING, vol. 13, 2011, pages 89-95, XP027575830,
- CHOI ET AL: "Effects of molten-salt/ionic-liquid mixture on extraction of docosahexaenoic acid (DHA)-rich lipids from Aurantiochytrium sp. KRS101", BIOPROCESS AND BIOSYSTEMS ENGINEERING, vol. 37, 10 May 2014 (2014-05-10), pages 2199-2204, XP035405788,
- CHANG ET AL: "Australian thraustochytrids: potential production of dietary long-chain omega-3 oils using crude glycerol", JOURNAL OF FUNCTIONAL FOODS, vol. 19, 7 February 2015 (2015-02-07), pages 810-820, XP029307938,

### SEQUENCE LISTING

<110> SYNTHETIC GENOMICS, INC. RADAKOVITS, Randor R. CHAMPAGNE, Michele L. TOLEDO, Gerardo V. WARD, Jillian
<120> NOVEL LABYRINTHULOMYCETE STRAINS FOR PRODUCING DOCOSAHEXANOIC ACID
<130> SGI1710-2WO
<150> US 62/002,107
   <151> 2014-05-22
<160> 12
<170> PatentIn version 3.5
<210> 1
   <211> 724
   <212> DNA
   <213> Artificial Sequence
<220>
   <223> Aurantiochytrium sp. 18S rDNA Fragment 1 from strain WH-05554
<220>
   <221> misc_feature
   <222> (641)..(641)
   <223> n is a, c, g, or t
<400> 1
   tccgcaggtt cacctacgga aaccttgtta cgacttcacc ttcctctaaa caataagatt      60
   cacccgagtt ctgcctctgt ccaaaaatta atccaaacag aaacatccca tggtttcatc     120
   ggaccgttca atcggtaggt gcgacgggcg gtgtgtacaa agggcaggga cgtattcaat     180
   gcaagctgat gacttgcgtt tactaggaat tcctcgttgg agattaataa ttgcaaaaat     240
   ctagccccag cacgatgagc gttccaagga ttagccaggc cttccgacca agcactcaat     300
   tccattaaaa tagaattaaa acccgatgaa cccatcagtg tagcgcgcgt gcggcccaga     360
   acatctaagg gcatcacaga cctgttattg cctcgaactt cctgcccgta aaccggacat     420
   gtccctctaa gaagttaaaa acgtactatg ttgccatacc acgcactatt tagtaggccg     480
   aggtctcgtt cgttaacgga attaaccaga caaatcactc caccaactaa gaacggccat     540
   gcaccaccac ccatagaatc atgaaagagc tctcaatctg tcaatcctac ctatgtctgg     600
   acctggtaag ttttcccgtg ttgagtcaaa ttaagccgca ngctccactc ctggtggtgc     660
   ccttccgtca attcctttaa gtttcagcct tgcgaccata ctccccccgg aacccaaaga     720
   cttt                                                                  724
<210> 2
   <211> 955
   <212> DNA
   <213> Artificial Sequence
<220>
   <223> Aurantiochytrium sp. 18S rDNA Fragment 2 from strain WH-05554
<220>
   <221> misc_feature
   <222> (124)..(124)
   <223> n is a, c, g, or t
<400> 2
   tctagcccca gcacgatgag cgttccaagg attagccagc cttccgacca agcactcaat    60
   tccaaaaaat agaattaaaa cccgatgaac ccatcagtgt agcgcgcgtg cggcccagaa   120
   catntaaggg catcacagac ctgttattgc ctcgaacttc ctgcccgtaa accggacatg   180
   tccctctaag aagtaaaaac gtactatgtt gccataccac gcactattta gtaggccgag   240
   gtctcgttcg ttaacggaat taaccagaca aatcactcca ccaactaaga acggccatgc   300
   accaccaccc atagaatcat gaaagagctc tcaatctgtc aatcctacct atgtctggac   360
   ctggtaagtt ttcccgtgtt gagtcaaatt aagccgcagg ctccactcct ggtggtgccc   420
   ttccgtcaat tcctttaagt ttcagccttg cgaccatact ccccccggaa cccaaagact   480
   ttgatttctc atgtgctgct gctgaggccc atagaataaa gcacccaaca atcgcaagtc   540
   ggcatcgttt acggtctaga ctacgatggt atctaatcat cttcgatccc cagactttcg   600
   ttcttgatta atgaaaacat gcttggtaaa tgccttcgct ctagttcgtc tttcggaaat   660
   ccaagaattt cacctctagc tcctaaatac gaataccccc aactgttcct attaaccatt   720
   actcaggcgt gcaaaccaac aaaatagcac ccaagtccta tcttatcatc ccataataaa   780
   cataccggtc atacgacctg cttggaacac tctgctttga ttacagtgaa agatttctca   840
   tcaataaaga aaagaaaaag atggccaagg caacacagac aatcaatccc cattcaggga   900
   aagcaccggt cgcccatgcc agaaattcaa ctacgagctt tttaactgca acaac        955
<210> 3
   <211> 902
   <212> DNA
   <213> Artificial Sequence
<220>
   <223> Aurantiochytrium sp. 18S rDNA Fragment 3 from strain WH-05554
<400> 3
   tttgatttct catgtgctgc tgctgaggcc catataaaaa agcacccaac aatcgcaagt    60
   cggcatcgtt tacggtctag actacgatgg tatctaatca tcttcgatcc ccagactttc   120
   gttcttgatt aatgaaaaca tgcttggtaa atgccttcgc tctagttcgt ctttcggaaa      180
   tccaagaatt tcacctctag ctcctaaata cgaatacccc caactgttcc tattaaccat      240
   tactcaggcg tgcaaaccaa caaaatagca cccaagtcct atcttatcat cccataataa      300
   acataccggt catacgacct gcttggaaca ctctgctttg attacagtga aagatttctc      360
   cccaataaag aaaagaaaaa gatggccaag gcaacacaga caatcaatcc ccattcaggg      420
   aaagcaccgg tcgcccatgc cagaaattca actacgagct ttttaactgc aacaacttta      480
   gcatatgctt ctggagctgg aattaccgcg gctgctggca ccagacttgc cctccagttg      540
   atcctcgatg agggttttac attgctctca ttccgatagc aaaacgcata cacgcttcgc      600
   atcgatattt ctcgtcacta cctcgtggag tccacagtgg gtaatttacg cgcctgctgc      660
   tatccttgga tatggtagcc gtctctcagg ctccctctcc ggagtcgagc cctaactctc      720
   cgtcacccgt tatagtcacc gtagtccaat acactaccgt cgacaactga tggggcagaa      780
   actcaaacga ttcatcgacc aaaaatagtc aatctgctca attatcatga ttcaccaata      840
   aaatcggctt caatctaata agtgcagccc catacagggc tcttacagca tgtattattt      900
   cc                                                                     902
<210> 4
   <211> 711
   <212> DNA
   <213> Artificial Sequence
<220>
   <223> Aurantiochytrium sp. 18S rDNA Fragment 4 from strain WH-05554
<400> 4
   actctgcttt gattacagtg aaagatctca taccaaaaaa tagcatgaga aagatggcca 60
   aggcaacaca gacaatcaat ccccattcag ggaaagcacc ggtcgcccat gccagaaatt 120
   caactacgag ctttttaact gcaacaactt tagcatatgc ttctggagct ggaattaccg 180
   cggctgctgg caccagactt gccctccagt tgatcctcga tgagggtttt acattgctct 240
   cattccgata gcaaaacgca tacacgcttc gcatcgatat ttctcgtcac tacctcgtgg 300
   agtccacagt gggtaattta cgcgcctgct gctatccttg gatatggtag ccgtctctca 360
   ggctccctct ccggagtcga gccctaactc tccgtcaccc gttatagtca ccgtagtcca 420
   atacactacc gtcgacaact gatggggcag aaactcaaac gattcatcga caaaaaatgt 480
   caatctgctc aattatcatg attcaccaat aaaatcggct tcaatctaat aagtgcagcc 540
   ccgtacaggg ctcttacagc atgtattatt tccagaatta ctgcaggtat ccatataaaa 600
   gaaactaccg aagaaattat tactgatata atgagccgtt cgcagtctca cagtacaatc 660
   gcttatactt acacatgcat ggcttaatct ttgagacaag catatgacta c 711
<210> 5
   <211> 672
   <212> DNA
   <213> Artificial Sequence
<220>
   <223> Aurantiochytrium sp. 18S rDNA Fragment 1 from strain NH-05783
<220>
   <221> misc_feature
   <222> (638)..(638)
   <223> n is a, c, g, or t
<400> 5
   tccgcaggtt cacctacgga aaccttgtta cgacttcacc ttcctctaaa caataagatt 60
   cacccgagtt ctgcctctgt ccaaaaatca atccaaacag aaacatccca tggtttcatc 120
   ggaccgttca atcggtaggt gcgacgggcg gtgtgtacaa agggcaggga cgtattcaat 180
   gcaagctgat gacttgcgtt tactaggaat tcctcgttgg agattaataa ttgcaaaaat 240
   ctagccccag cacgatgagc gttccaagga ttagccaggc cttccgacca agcactcaat 300
   tccaaaaatg aaattaaaac ccgatgaacc catcagtgta gcgcgcgtgc ggcccagaac 360
   atctaagggc atcacagacc tgttattgcc tcgaacttcc tgcccgtaaa ccggacatgt 420
   ccctctaaga agtaaaaacg tactatgttg ccataccacg cactatttag taggccgagg 480
   tctcgttcgt taacggaatt aaccagacaa atcactccac caactaagaa cggccatgca 540
   ccaccaccca tagaatcatg aaagagctct caatctgtca atcctaccta tgtctggacc 600
   tggtaagttt tcccgtgttg agtcaaatta agccgcangc tccactcctg gtggtgccct 660
   tccgtcaatt cc 672
<210> 6
   <211> 897
   <212> DNA
   <213> Artificial Sequence
<220>
   <223> Aurantiochytrium sp. 18S rDNA Fragment 2 from strain NH-05783
<400> 6
   cactcaattc caaaaatgaa attaaaaccc gatgaaccca tcagtgtagc gcgcgtgcgg 60
   cccagaacat ctaagggcat cacagacctg ttattgcctc gaacttcctg cccgtaaacc 120
   ggacatgtcc ctctaagaag taaaaacgta ctatgttgcc ataccacgca ctatttagta 180
   ggccgaggtc tcgttcgtta acggaattaa ccagacaaat cactccacca actaagaacg 240
   gccatgcacc accacccata gaatcatgaa agagctctca atctgtcaat cctacctatg 300
   tctggacctg gtaagttttc ccgtgttgag tcaaattaag ccgcaggctc cactcctggt 360
   ggtgcccttc cgtcaattcc tttaagtttc agccttgcga ccatactccc cccggaaccc 420
   aaagactttg atttctcatg tgctgctgct gaggcccata aataaagcac ccaacaatcg 480
   caagtcggca tcgtttacgg tctagactac gatggtatct aatcatcttc gatccccaga 540
   ctttcgttct tgattaatga aaacatgctt ggtaaatgcc ttcgctctag ttcgtctttc 600
   ggaaatccaa gaatttcacc tctagctcct aaatacgaat acccccaact gttcctatta 660
   accattactc aggcgtgcaa accaacaaaa tagcacccaa gtcctatctt atcatcccat 720
   aataaacata ccggtcatac gacctgcttg gaacactctg ctttgattac agtgaaagat 780
   ttctccccta taaagaaaag aaaaagatgg ccaaggcaac acagacaatc aatccccatt 840
   cagggaaagc accggtcgcc catgccagaa attcaactac gagcttttta actgcaa 897
<210> 7
   <211> 975
   <212> DNA
   <213> Artificial Sequence
<220>
   <223> Aurantiochytrium sp. 18S rDNA Fragment 3 from strain NH-05783
<220>
   <221> misc_feature
   <222> (912)..(913)
   <223> n is a, c, g, or t
<400> 7
   tttgatttct catgtgctgc tgctgaggcc cataaataaa gcacccaaca atcgcaagtc      60
   ggcatcgttt acggtctaga ctacgatggt atctaatcat cttcgatccc cagactttcg     120
   ttcttgatta atgaaaacat gcttggtaaa tgccttcgct ctagttcgtc tttcggaaat     180
   ccaagaattt cacctctagc tcctaaatac gaataccccc aactgttcct attaaccatt     240
   actcaggcgt gcaaaccaac aaaatagcac ccaagtccta tcttatcatc ccataataaa     300
   cataccggtc atacgacctg cttggaacac tctgctttga ttacagtgaa agatttctcc     360
   cctataaaga aaagaaaaag atggccaagg caacacagac aatcaatccc cattcaggga     420
   aagcaccggt cgcccatgcc agaaattcaa ctacgagctt tttaactgca acaactttag     480
   catatgcttc tggagctgga attaccgcgg ctgctggcac cagacttgcc ctccagttga     540
   tcctcgatga gggttttaca ttgctctcat tccgatagca aaacgcatac acgcttcgca     600
   tcgatatttc tcgtcactac ctcgtggagt ccacagtggg taatttacgc gcctgctgct     660
   atccttggat atggtagccg tctctcaggc tccctctccg gagtcgagcc ctaactctcc     720
   gtcacccgtt atagtcaccg tagtccaata cactaccgtc gacaactgat ggggcagaaa     780
   ctcaaacgat tcatcgacaa aaatagtcaa tctgctcaat tatcatgatt caccaataaa     840
   atcggcttca atctaataag tgcagcccca tacagggctc ttacagcatg tattatttcc     900
   agaattactg cnntatccat ataaaagaaa ctaccgaaga aattattact gatataatga     960
   gccgttcgca gtctc                                                      975
<210> 8
   <211> 637
   <212> DNA
   <213> Artificial Sequence
<220>
   <223> Aurantiochytrium sp. 18S rDNA Fragment 4 from strain NH-05783
<400> 8
   atcaatcccc attcagggaa agcaccggtc gcccatgcca gaaattcaac tacgagcttt 60
   ttaactgcaa caactttagc atatgcttct ggagctggaa ttaccgcggc tgctggcacc 120
   agacttgccc tccagttgat cctcgatgag ggttttacat tgctctcatt ccgatagcaa 180
   aacgcataca cgcttcgcat cgatatttct cgtcactacc tcgtggagtc cacagtgggt 240
   aatttacgcg cctgctgcta tccttggata tggtagccgt ctctcaggct ccctctccgg 300
   agtcgagccc taactctccg tcacccgtta tagtcaccgt agtccaatac actaccgtcg 360
   acaactgatg gggcagaaac tcaaacgatt catcgactaa aaaagtcaat ctgctcaatt 420
   atcatgattc accaataaaa tcggcttcaa tctaataagt gcagccccat acagggctct 480
   tacagcatgt attatttcca gaattactgc aggtatccat ataaaagaaa ctaccgaaga 540
   aattattact gatataatga gccgttcgca gtctcacagt acaatcgctt atacttacac 600
   atgcatggct taatctttga gacaagcata tgactac 637
<210> 9
   <211> 699
   <212> DNA
   <213> Artificial Sequence
<220>
   <223> Aurantiochytrium sp. 18S rDNA Fragment 1 from strain NH-06161
<220>
   <221> misc_feature
   <222> (639)..(639)
   <223> n is a, c, g, or t
<400> 9
   tccgcaggtt cacctacgga aaccttgtta cgacttcacc ttcctctaaa caataagatt 60
   cacccgagtt ctgcctctgt ccaaaaatca atccaaacag aaacatccca tggtttcatc 120
   ggaccgttca atcggtaggt gcgacgggcg gtgtgtacaa agggcaggga cgtattcaat 180
   gcaagctgat gacttgcgtt tactaggaat tcctcgttgg agattaataa ttgcaaaaat 240
   ctagccccag cacgatgagc gttccaagga ttagccaggc cttccgacca agcactcaat 300
   tccaaaaatt gaaattaaaa cccgatgaac ccatcagtgt agcgcgcgtg cggcccagaa 360
   catctaaggg catcacagac ctgttattgc ctcgaacttc ctgcccgtaa accggacatg 420
   tccctctaag aagtaaaaac gtactatgtt gccataccac gcactattta gtaggccgag 480
   gtctcgttcg ttaacggaat taaccagaca aatcactcca ccaactaaga acggccatgc 540
   accaccaccc atagaatcat gaaagagctc tcaatctgtc aatcctacct atgtctggac 600
   ctggtaagtt ttcccgtgtt gagtcaaatt aagccgcang ctccactcct ggtggtgccc 660
   ttccgtcaat tcctttaagt ttcagccttg cgaccatac 699
<210> 10
   <211> 907
   <212> DNA
   <213> Artificial Sequence
<220>
   <223> Aurantiochytrium sp. 18S rDNA Fragment 2 from strain NH-06161
<400> 10
   cgaccaagca ctcaattcca aaaattgaaa ttaaaacccg atgaacccat cagtgtagcg 60
   cgcgtgcggc ccagaacatc taagggcatc acagacctgt tattgcctcg aacttcctgc 120
   ccgtaaaccg gacatgtccc tctaagaagt aaaaacgtac tatgttgcca taccacgcac 180
   tatttagtag gccgaggtct cgttcgttaa cggaattaac cagacaaatc actccaccaa 240
   ctaagaacgg ccatgcacca ccacccatag aatcatgaaa gagctctcaa tctgtcaatc 300
   ctacctatgt ctggacctgg taagttttcc cgtgttgagt caaattaagc cgcaggctcc 360
   actcctggtg gtgcccttcc gtcaattcct ttaagtttca gccttgcgac catactcccc 420
   ccggaaccca aagactttga tttctcatgt gctgctgctg aggcccataa aaaagcaccc 480
   aacaatcgca agtcggcatc gtttacggtc tagactacga tggtatctaa tcatcttcga 540
   tccccagact ttcgttcttg attaatgaaa acatgcttgg taaatgcctt cgctctagtt 600
   cgtctttcgg aaatccaaga atttcacctc tagctcctaa atacgaatac ccccaactgt 660
   tcctattaac cattactcag gcgtgcaaac caacaaaata gcacccaagt cctatcttat 720
   catcccataa taaacatacc ggtcatacga cctgcttgga acactctgct ttgattacag 780
   tgaaagatct cataccaaaa tagcatgaga aagatggcca aggcaacaca gacaatcaat 840
   ccccattcag ggaaagcacc ggtcgcccat gccagaaatt caactacgag ctttttaact 900
   gcaacaa 907
<210> 11
   <211> 917
   <212> DNA
   <213> Artificial Sequence
<220>
   <223> Aurantiochytrium sp. 18S rDNA Fragment 3 from strain NH-06161
<400> 11
   aagactttga tttctcatgt gctgctgctg aggcccatat aataaagcac ccaacaatcg        60
   caagtcggca tcgtttacgg tctagactac gatggtatct aatcatcttc gatccccaga       120
   ctttcgttct tgattaatga aaacatgctt ggtaaatgcc ttcgctctag ttcgtctttc       180
   ggaaatccaa gaatttcacc tctagctcct aaatacgaat acccccaact gttcctatta       240
   accattactc aggcgtgcaa accaacaaaa tagcacccaa gtcctatctt atcatcccat       300
   aataaacata ccggtcatac gacctgcttg gaacactctg ctttgattac agtgaaagat       360
   ctcataccga aaacagcatg agaaagatgg ccaaggcaac gcagacaatc aatccccatt       420
   cagggaaagc accggtcgcc catgccagaa attcaactac gagcttttta actgcaacaa       480
   ctttagcata tgcttctgga gctggaatta ccgcggctgc tggcaccaga cttgccctcc       540
   agttgatcct cgatgagggt tttacattgc tctcattccg atagcaaaac gcatacacgc       600
   ttcgcatcga tatttctcgt cactacctcg tggagtccac agtgggtaat ttacgcgcct       660
   gctgctatcc ttggatatgg tagccgtctc tcaggctccc tctccggagt cgagccctaa       720
   ctctccgtca cccgttatag tcaccgtagt ccaatacact accgtcgaca actgatgggg       780
   cagaaactca aacgattcat cgactcaaaa agtcaatctg ctcaattatc atgattcacc       840
   aataaaatcg gcttcaatct aataagtgca gccccataca gggctcttac agcatgtatt       900
   atttccagaa ttactgc                                                      917
<210> 12
   <211> 708
   <212> DNA
   <213> Artificial Sequence
<220>
   <223> Aurantiochytrium sp. 18S rDNA Fragment 4 from strain NH-06161
<400> 12
   tctgctttga ttacagtgaa aagatctcat accaaaatag catgagaaag atggccaagg      60
   caacacagac aatcaatccc cattcaggga aagcaccggt cgcccatgcc agaaattcaa     120
   ctacgagctt tttaactgca acaactttag catatgcttc tggagctgga attaccgcgg     180
   ctgctggcac cagacttgcc ctccagttga tcctcgatga gggttttaca ttgctctcat     240
   tccgatagca aaacgcatac acgcttcgca tcgatatttc tcgtcactac ctcgtggagt     300
   ccacagtggg taatttacgc gcctgctgct atccttggat atggtagccg tctctcaggc     360
   tccctctccg gagtcgagcc ctaactctcc gtcacccgtt atagtcaccg tagtccaata     420
   cactaccgtc gacaactgat ggggcagaaa ctcaaacgat tcatcgacca aaaaagtcaa     480
   tctgctcaat tatcatgatt caccaataaa atcggcttca atctaataag tgcagcccca     540
   tacagggctc ttacagcatg tattatttcc agaattactg caggtatcca tataaaagaa     600
   actaccgaag aaattattac tgatataatg agccgttcgc agtctcacag tacaatcgct     660
   tatacttaca catgcatggc ttaatctttg agacaagcat atgactac                  708

## Description

### BACKGROUND

Long-chain omega-3 fatty acids are an essential part of the human diet that are currently derived mainly from fish oils. Due to problems with overfishing as well as heavy metal contamination of fish stocks, there is a need for an alternative sustainable source of omega-3 fatty acids such as eicosapentaenoic acid (EPA) and docosahexaenoic acid (DHA) that have demonstrated health benefits in humans.

Chytrids (eukaryotic marine microorganisms of the labyrinthylomycetes class) are currently used as a source of DHA but the cost of chytrid-produced DHA is currently high in comparison with fish-derived DHA. Strains having enhanced rates of DHA production can be used to reduce the cost of producing DHA. Chang et al, Marine Biotechnology, (2014), vol. 16, pages 396 - 411 describes a comparison of Thraustochytrids Aurantiochytrium sp., Schizochytrium sp., Thraustochytrium sp., and Ulkenia sp. for production of biodiesel, long-chain omega-3 oils, and exopolysaccharide. US 2010/0285105 describes oil-producing microbes and methods of modification thereof. Chang et al, Journal of Functional Foods, (February 2015), pages 810 - 820 describes potential production of dietary long-chain omega-3 oils using crude glycerol in Australian thraustochytrids.

### SUMMARY

Described herein are mutant strains of the labyrinthulomycete class of microorganisms useful in producing docosahexaenoic acid (DHA) and myristic acid, and oils produced by such strains. The strains described herein can also be used to isolate derivative strains, including strains having new or improved traits, such as enhanced lipid production, growth, nutrient utilization, or chemical tolerance as compared to a progenitor strain. Also described herein are methods for isolating derivation stains having enhanced traits, such as but not limited to rates of lipid production, with respect to progenitor strains, for example via mutagenesis and/or selection in a cytostat or chemostat, optionally in the presence of a selective agent. The embodiments of the invention for which protection is sought are defined by the claims. In a first aspect, the invention provides a microbial oil produced by a mutant labyrinthulomycete microrganism, wherein the labyrinthulomycete microorganism is a strain of Thraustochytrium, Schizochytrium, or Aurantiochytrium, wherein the microbial oil comprises at least 35% of the total fatty acids as docosahexaenoic acid (DHA) and at least 25% of the total fatty acids as myristic acid, and wherein the microorganism has been subjected to UV treatment. The ratio of DHA to DPA may be at least 3.5 : 1. 10% or less of the total fatty acids may comprise docosapentaenoic acid (DPA); the total fatty acids may comprise less than 2% arachidonic acid (ARA); and/or the total fatty acids may comprise less than 1% eicosapentaenoic acid (EPA). The microbial oil may be a crude oil or a refined oil.
In a second aspect, the invention provides a product comprising a microbial oil according to the first aspect. The product may be a food product, an animal feed product, a cosmetic product, or a pharmaceutical product.
In a third aspect, the inveition provides a mutant *Thraustochytrium*, *Schizochytrium*, or *Aurantiochytrium* microorganism that produces a microbial oil according to the first aspect, wherein the microorganism has been subjected to UV treatment. The mutant microorganism may produce DHA in a small scale culture at a rate of at least 150 mg/L/h. The mutant microorganism may produce at least 20% more DHA and at least 100% more myristic acid as a percent of total fatty acids than the wild type strain from which it is derived. The mutant Thraustochytrium, Schizochytrium, or Aurantiochytrium microorganism may comprise an 18S rDNA sequence having at least 98% identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, and SEQ ID NO:12. The mutant microorganism may comprise an 18S rDNA sequence having at least 98% identity to: a sequence selected from SEQ ID NO:5 and SEQ ID NO:9; a sequence selected from SEQ ID NO:6 and SEQ ID NO:10; a sequence selected from SEQ ID NO:7 and SEQ ID NO:11; and a sequence selected from SEQ ID NO:8 and SEQ ID NO:12. The mutant microorganism may comprise an 18S rDNA sequence having at least 98.5% identity to: a sequence selected from SEQ ID NO:5 and SEQ ID NO:9; a sequence selected from SEQ ID NO:6 and SEQ ID NO:10; a sequence selected from SEQ ID NO:7 and SEQ ID NO:11; and a sequence selected from SEQ ID NO:8 and SEQ ID NO:12. In a fourth aspect, the invention provides a microbial biomass comprising the mutant microorganism of the third aspect. In a fifth aspect, the inveition provides a product comprising a microbial biomass according to the fourth aspect.

Also described herein are labyrinthulomycete microorganisms useful in producing DHA. These are not part of the scope unless they
fulfil the conditions of the claims. The labyrinthulomycete microorganisms can be microorganisms that have been deposited with the Agricultural Research Service Culture Collection located at 1815 N. University Street, Peoria, IL 61604, USA (NRRL) on April 4, 2013 as NRRL-50836 (strain NH-05783) and NRRL-50837 (strain NH-06161); or can be microorganisms of strains derived from either of these deposited strains. A strain as provided herein can include an 18S rRNA gene that includes sequence having at least 95%, 96%, 97%, 97.5%. 98%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8%, or 99.9% identity to SEQ ID NO:1, SEQ ID NO:2, SEQ ID NO:3, and/or SEQ ID NO:4; or can include an 18S rRNA gene that includes sequence having at least 95%, 96%, 97%, 97.5%. 98%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8%, or 99.9% identity to SEQ ID NO:5, SEQ ID NO:6, SEQ ID NO:7, and/or SEQ ID NO:8; or can include an 18S rRNA gene that includes sequence having at least 95%, 96%, 97%, 97.5%. 98%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8%, or 99.9% identity to SEQ ID NO:9, SEQ ID NO:10, SEQ ID NO:11, and/or SEQ ID NO:12.

Also described herein are isolated microorganisms of strains derived from NH-05783 or NH-06161 by any method, including but not limited to subculturing with or without selection for a trait of interest, chemostat or cytostat selection, mutagenesis, genetic engineering, or any combination thereof.

In various instances, the strains described herein, including NRRL-50836 (strain NH-05783) and NRRL-50837 (strain NH-06161) and strains derived therefrom can produce at least 25% of fatty acids as DHA. For example, at least 25%, at least 30%, at least 35%, or at least 40% of fatty acids produced by a strain, including a derived strain *(e.g.,* a mutant or variant strain), can be DHA. The strains can produce DHA at a rate of at least 40 mg/L/h, at least 45 mg/L/h, at least 50 mg/L/h, at least 100 mg/L/h, at least 130 mg/L/h, at least 160 mg/L/h, at least 190 mg/L/h, or at least 200mg/L/h when grown in small scale batch culture. In some instances, the strains can produce DHA at a rate of at least 100 mg/L/h, at least 150 mg/L/h, at least 200 mg/L/h, at least 250 mg/L/h, at least 300 mg/L/h, or at least 350 mg/L/h, when grown as 25, 50, 100, 200, 400, or 500 ml batch cultures in shake flasks. In some instances, the strains can produce DHA at a rate of at least 100 mg/L/h, at least 150 mg/L/h, at least 200 mg/L/h, at least 250 mg/L/h, at least 300 mg/L/h, at least 350 mg/L/h, at least 400 mg/L/h, at least 450 mg/L/h, at least 500 mg/L/h, at least 600 mg/L/h, at least 700 mg/L/h, or at least 800 mg/L/h, when grown in fermentation cultures of at least one liter. The strains disclosed herein and strains derived therefrom can produce lipid in which the ratio of DHA to docosapentaenoic acid (C22:5n6; DPA) is at least 3.5 to 1, or at least about 4.0 to 1. The percentage of fatty acids as docosapentaenoic acid (DPA) produced by a microorganism as provided herein can be, for example, less than 12% or less than 10%. Strains NH-05783, NH-06161, and derivatives thereof can produce an oil in which at least 25%, 30%, or 35% of the fatty acids are DHA, and at least 10%, at least 12%, at least 15%, at least 20%, at least 25%, or at least 30% of the fatty acids are myristic acid. In some examples, less than about 5%, less than about 3%, or less than about 2% of the fatty acids are eicosapentaenoic acid (EPA). Additionally, an oil produced by a strain provided herein or a derivative thereof can have less than 2%, less than 1%, or less than 0.5% of fatty acids as arachidonic acid (ARA).

Also described herein are isolated microorganisms and mutant microorganisms of the labyrinthulomycete class and mutants or variants derived therefrom, where the microorganisms have an 18S rRNA gene comprising a sequence having at least least 95%, 96%, 97%, 97.5%. 98%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8%, or 9.99% identity to SEQ ID NO:1, SEQ ID NO:2, SEQ ID NO:3, and SEQ ID NO:4. The strain can be useful in the production of omega-3 fatty acids, for example, DHA, and can produce lipid in which at least 20%, at least 25%, at least 30%, at least 35%, at least 40%, or at least 45% of the fatty acids of the produced lipid are DHA. The strains can in some examples be characterized as belonging to the genus *Thraustochytrium, Schizochytrium*, or *Aurantiochytrium* and can be mutants of wild type or native strains, in which the mutants produce at least 20%, at least 30%, at least 40%, or at least 50% more DHA than the wild type strain from which they are derived. Alternatively or in addition, the strains can in some examples be characterized as belonging to the genus *Thraustochytrium, Schizochytrium*, or *Aurantiochytrium* and can be mutants of wild type or native strains, in which the mutants produce at least 50%, at least 100%, at least 200%, or at least 300% more myristic acid than the wild type strain from which they are derived. For example, a mutant microorganism can produce at least 10%, at least 15%, at least 20%, at least 25%, or at least 30% of its total fatty acids as myristic acid. The strains can additionally produce lipid in which the ratio of DHA to DPA is at least about 3.5 to 1 or at least about 4.0 to 1. The strain can in some examples produce DHA at a rate of at least 40 mg/L/h, at least 45 mg/L/h, at least 50 mg/L/h, at least 100 mg/L/h, at least 130 mg/L/h, at least 160 mg/L/h, at least 190 mg/L/h, or at least 200mg/L/h when grown in small scale, twelve to twenty-four hour batch culture, for example a fourteen hour or twenty-three hour batch culture.

Also described herein are isolated microorganisms and mutant microorganisms of the labyrinthulomycete class, where the microorganisms have an 18S rRNA gene comprising a sequence having at least 95%, 96%, 97%, 97.5%. 98%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8%, or 9.99% identity to SEQ ID NO:5, SEQ ID NO:6, SEQ ID NO:7, and SEQ ID NO:8. The strains can be useful in the production of omega-3 fatty acids, for example, DHA, and can produce lipid in which at least 20%, at least 25%, at least 30%, at least 35%, at least 40%, or at least 45% of the fatty acids of the produced lipid are DHA. The strains can in some examples be characterized as belonging to the genus *Thraustochytrium, Schizochytrium*, or *Aurantiochytrium* and can be mutants of wild type or native strains, in which the mutants produce at least 20%, at least 30%, at least 40%, or at least 50% more DHA than the wild type strain from which they are derived. Alternatively or in addition, the strains can in some examples be characterized as belonging to the genus *Thraustochytrium, Schizochytrium*, or *Aurantiochytrium* and can be mutants of wild type or native strains, in which the mutants produce at least 50%, at least 100%, at least 200%, or at least 300% more myristic acid than the wild type strain from which they are derived. For example, a mutant microorganism can produce at least 10%, at least 15%, at least 20%, at least 25%, or at least 30% of its total fatty acids as myristic acid. The strains can additionally produce lipid in which the ratio of DHA to DPA is at least about 3.5 to 1 or at least about 4.0 to 1. The strain can in some examples produce DHA at a rate of at least 40 mg/L/h, at least 45 mg/L/h, at least 50 mg/L/h, at least 100 mg/L/h, at least 130 mg/L/h, at least 160 mg/L/h, at least 190 mg/L/h, or at least 200mg/L/h when grown in small scale, twelve to twenty-four hour batch culture, for example a fourteen hour or twenty-three hour batch culture.

Also described herein are isolated microorganisms and mutant microorganisms of the labyrinthulomycete class and mutants or variants derived therefrom, where the microorganisms have an 18S rRNA gene comprising a sequence having at least 95%, 96%, 97%, 97.5%. 98%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8%, or 99.9% identity to SEQ ID NO:9, SEQ ID NO:10, SEQ ID NO:11, and SEQ ID NO:12. The strains can be useful in the production of omega-3 fatty acids, for example, DHA, and can produce lipid in which at least 20%, at least 25%, at least 30%, at least 35%, at least 40%, or at least 45% of the fatty acids of the produced lipid are DHA. The strains can in some examples be characterized as belonging to the genus *Thraustochytrium, Schizochytrium*, or *Aurantiochytrium* and can be mutants of wild type or native strains, in which the mutants produce at least 20%, at least 30%, at least 40%, or at least 50% more DHA than the wild type strain from which they are derived. Alternatively or in addition, the strains can in some examples be characterized as belonging to the genus *Thraustochytrium, Schizochytrium*, or *Aurantiochytrium* and can be mutants of wild type or native strains, in which the mutants produce at least 50%, at least 100%, at least 200%, or at least 300% more myristic acid than the wild type strain from which they are derived. For example, a mutant microorganism can produce at least 10%, at least 15%, at least 20%, at least 25%, or at least 30% of its total fatty acids as myristic acid. The strains can additionally produce lipid in which the ratio of DHA to DPA is at least about 3.5 to 1 or at least about 4.0 to 1. The strain can in some examples produce DHA at a rate of at least 40 mg/L/h, at least 45 mg/L/h, at least 50 mg/L/h, at least 100 mg/L/h, at least 130 mg/L/h, at least 160 mg/L/h, at least 190 mg/L/h, or at least 200mg/L/h when grown in small scale, twelve to twenty-four hour batch culture, for example a fourteen hour or twenty-three hour batch culture.

Also described is a mutant microorganism that produces an increased amount of myristic acid as a percentage of total fatty acids produced with respect to the strain from which it is derived. For example, the mutant can produce at least 20% more, at least 30% more, at least 40% more, at least 50% more, at least 70% more, or at least 100% more myristic acid as a percentage of total fatty acids produced than the strain from which the mutant is derived. The mutant in some examples can produce at least 2-fold, at least 3-fold, at least 4-fold, at least 5-fold, or at least 6-fold myristic acid as a percentage of total fatty acids produces as compared to the strain from which the mutant was derived. The mutant microorganism can produce, for example, at least 10%, at least 15%, at least 20%, at least 25%, or at least 30% of its fatty acids as myristic acid. In some examples, the microorganism is of the labyrinthulomycete class. In some examples, the microorganism is a species of *Thraustochytrium, Schizochytrium*, or *Aurantiochytrium.* The mutant microorganism can additionally produce more DHA as a percent of total fatty acids with respect to the strain from which it is derived.Also provided herein is a mutant microorganism that produces more myristic acid and more DHA as a percentage of total fatty acids than is produced by the strain from which the mutant is derived. For example, in addition to producing at least 50% more or at least 100% more myristic acid as a percentage of total fatty acids produced as compared with a wild type or progenitor strain, the mutant can produce at least 20% more, at least 30% more, at least 40% more, at least 50% more, at least 70% more, or at least 100% more DHA as a as a percentage of total fatty acids produced than the strain from which the mutant is derived. The mutant microorganism can produce, for example, at least 10%, at least 15%, at least 20%, at least 25%, at least 30%, at least 35% or at least 39% of its fatty acids as DHA and at least 10%, at least 15%, at least 20%, at least 25%, or at least 30%, of its fatty acids as myristic acid. A mutant labyrinthulomycete strain in some examples produces DHA in a small scale batch fermentation culture at a rate of at least 150 mg/L/h. For example, a mutant labyrinthulomycete microorganism as provided herein can produce DHA in a small scale culture at a rate of at least 170 mg/L/h, at a rate of at least 190 mg/L/h, or at a rate of at least 200 mg/L/h. In various examples, the total fatty acids produced by a mutant microorganism as provided herein comprise less than 2% ARA, for example, less than 1% ARA, and may comprise less than 3% EPA, for example, less than 1% EPA. The ratio of DHA to DPA produced by the mutant microorganism can in some examples be at least 4:1.

Such mutant strains can be strains of, for example, a genus such as Labyrinthula, Labyrinthuloides, *Thraustochytrium, Schizochytrium, Aplanochytrium, Aurantiochytrium, Japonochytrium, Oblongichytrium, Diplophrys,* or *Ulkenia.* For example, a mutant strain as provided herein can be a strain of *Thraustochytrium, Schizochytrium,* or *Aurantiochytrium.* In some examples, the microorganism is a species of *Thraustochytrium, Schizochytrium*, or *Aurantiochytrium.* For example, a mutant strain that produces a higher percentage of fatty acids as DHA and a higher percentage of fatty acids as myristic acid can be an *Aurantiochytrium* or *Schizochytrium* strain having an 18S rDNA sequence at least 95%, at least 96%, at least 97%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, or at least 99.9% identical to any of SEQ ID NO:1, SEQ ID NO:2, SEQ ID NO:3, SEQ ID NO:4, SEQ ID NO:5, SEQ ID NO:6, SEQ ID NO:7, SEQ ID NO:8; SEQ ID NO:9, SEQ ID NO:10, SEQ ID NO:11, and SEQ ID NO:12.

The total fatty acids produced by a mutant labyrinthulomycete strain can include, for example, 10% or less of DPA, 9% or less of DPA, 8% or less of DPA, 7% or less of DPA, or 6% or less of DPA. The ratio of DHA to DPA in fatty acids produced by a mutant labyrinthulomycete strain as provided herein can be about 3.5 : 1 or higher, for example, about 4 : 1 or higher, for example, about 4.5 : 1 or higher, about 4.9 : 1 or higher, or about 5 : 1 or higher.

Also described are methods for isolating one or more derivatives or mutants of a microbial strain that have increased lipid production with respect to the progenitor microbial strain that include: culturing a microbial strain organism of interest in a cytostat or chemostat in the presence of at least one inhibitor of an enzyme or factor that participates in lipid metabolism, and isolating at least one derivative or mutant of the microbial strain that exhibits increased lipid production. Increased lipid production can be any of: an increased amount of lipid produced per culture volume, increased lipid produced as a percentage of cell dry weight, an increased amount of triglyceride (TAG) produced per culture volume, increased TAG produced as a percentage of cell dry weight, an increased amount of FAME produced per culture volume, increased FAME produced as a percentage of cell dry weight, an increased amount of one or more fatty acids (*e.g.,* one or more of a C8, C10, C12, C14, C16, or C18 fatty acid) produced per culture volume, increased amount of one or more fatty acids produced as a percentage of cell dry weight, increased amount of one or more fatty acids produced as a percentage of total fatty acids (FAME) produced, an increased amount of one or more PUFAs (*e.g.,* one or more of a ARA, EPA, or DHA) produced per culture volume, increased amount of one or more PUFAs produced as a percentage of cell dry weight, increased amount of one or more PUFAs produced as a percentage of total fatty acids (FAME) produced, an increased rate of production of any of total lipid, TAG, FAME, one or more fatty acids, or one or more PUFAs produced by the isolated strain. Colonies can be isolated after culturing a strain of interest in a cytostat or chemostat and the isolated derived strains grown from the isolated colonies can be tested for any of the above values. Derivative strains can also be tested and selected based on growth rate or biomass accumulation for example, including growth rate or biomass accumulation during a lipid production phase of a culture.

Prior to or following the step of selecting a microbial strain of interest with a lipid biosynthesis inhibitor, the strain can optionally be selected in a cytostat or chemostat that does not include a lipid biosynthesis inhibitor. Prior to selecting a microbial strain of interest in a cytostat or chemostat, the methods can also optionally include subjecting the strain to one or more mutagenesis protocols, which can use, for example, one or more chemical mutagens, UV light, or gamma irradiation. Further, the methods can optionally include selecting a microbial strain of interest sequentially in cytostat or chemostat culture using the same lipid biosynthesis inhibitor or a different lipid biosynthesis inhibitor. For example, the procedure of mutagenesis followed by chemostat or cytostat selection in the presence of at least one inhibitor of an enzyme or factor that participates in lipid metabolism can be performed one, two, three, or more times.

As also described herein, a microorganism can be treated with UV irradiation and screened for a trait, including but not limited to higher levels of total lipid, TAG, PUFAs, omega-3 fatty acids, or higher levels of one or more particular fatty acids, such as, for example, myristic acid, oleic acid, ARA, DHA, or EPA. UV treatment of a microorganism can be performed after mutagenesis of the strain, where the mutagenesis can employ UV or another mutagen. In some examples, the microorganism can be screened or selected for a trait of interest after mutagenesis and prior to UV treatment. Further, the microorganism can additionally or alternatively be screened or selected for a trait of interest after UV treatment. For example, a mutagenized microorganism that has been selected for increased lipid production can be subjected to UV irradiation and subsequently selected for or tested for yet higher levels of lipid production. A UV treated strain can optionally be selected in a chemostat or cytostat, with or without a compound that can select for a trait of interest, for example, a compound that inhibits lipid biosynthesis. Strains isolated after selection in the chemostat or cytostat can then be tested for enhancement of the trait of interest, for example, increased lipid, increased PUFAs, increased ARA, increased EPA, or increased DHA to isolate a derivative strain having increased level or rate of lipid, PUFA, ARA, EPA, or DHA production with respect to the mutagenized progenitor strain.

A microbial strain of interest that can be used in the methods for selecting derivatives having enhanced lipid production can be, for example, an algal strain, a bacterial strain, a fungal strain, or a heterokont strain. In some examples, the microbial strain is a strain of oleaginous yeast, such as, for example, a strain of *Candida, Cryptococcus, Lipomyces, Mortierella, Rhodosporidium, Rhodotortula, Trichosporon,* or *Yarrowia.* In some examples, the microbial strain is a strain of algae, such as, for example, a strain of *Botryococcus, Chlorella, Cyclotella, Dunaliella, Euglena, Hantzschia, Haematococcus Isochrisis, Monodus, Nannochloropsis, Neochloris, Nitzchia, Parietochloris, Pavlova,* or *Porphyridium.* In some examples, the microbial strain is a labyrinthulid strain or thraustochytrid strain. For example, the strain may be a species of Labyrinthula, Labyrinthuloides, *Thraustochytrium, Schizochytrium, Aplanochytrium, Aurantiochytrium, Japonochytrium, Oblongichytrium, Diplophrys,* or *Ulkenia.*

Also described herein are strains derived from wild type strains, laboratory strains, and manipulated strains, including genetically engineered or classically improved strains that have been selected in a cytostat or chemostat that includes an inhibitor of an enzyme that functions in lipid metabolism in the culture medium. Also described are strains derived using the methods herein that are subsequently genetically engineered or further improved by classical methods. The strains can be useful in producing polyunsaturated fatty acids such as, for example, DHA, and oils that include polyunsaturated fatty acids such as DHA. The strains can be used to produce biomass that can be used as a component of nutritional products for humans or animals.

Also described is a biomass comprising an isolated labyrinthulomycete strain as provided herein. In some examples, at least 20%, at least 25%, at least 30%, or at least 35% by weight of the fatty acids of the dried biomass of the isolated strain is DHA. Alternatively or in addition, at least 6%, at least 8%, at least 10%, at least 15%, at least 20%, or at least 25% by weight of the fatty acids of the dried biomass of the isolated strain can be myristic acid. In some examples, an isolated labyrinthulomycete biomass can comprise at least about 10%, at least about 20%, at least 30%, at least 40%, at least 50%, at least 60%, or at least 70% by weight of the dry cell weight of the biomass as fatty acids, and at least 20%, at least 25%, at least 30%, at least 40%, or at least 50% by weight of the fatty acids may be omega-3 fatty acids.

Also described is a microbial oil isolated from a microorganism as provided herein or a derivative thereof, for example a microbial oil from whole culture or biomass harvested after culturing any microorganism as provided herein. The microbial oil can include, for example, at least 20%, at least 25%, at least 30%, at least 35%, or at least 40% DHA. In some instances, the microbial oil can include at least 6%, at least 8%, at least 10%, at least 15%, at least 20%, at least 25%, or at least 30% of their fatty acids as myristate. In some instances, the ratio of docosahexaenoic acid to docosapentaenoic acid can be equal to or greater than 3.5:1 or greater than or equal to about 4:1. In some instances, the microbial oil comprises less than about 5%, less than 4%, less than 3%, less than 2%, less than 1%, or less than 0.5%, of EPA. In some instances, the microbial oil comprises less than about 2%, less than 1%, less than 0.5%, or undetectable amounts of ARA.

Also described is a food product, animal feed product, cosmetic, nutritional, therapeutic, or pharmaceutical comprising any one of the labyrinthulomycete microorganisms or biomasses described herein or mixtures thereof. Also described is a food product, cosmetic, or pharmaceutical composition for animals or humans comprising any of the microbial oils described herein. In some instances, the food product is an infant formula. In some instances, the food product is a milk, a beverage, a therapeutic drink, a nutritional drink, or a combination thereof. In some instances, the food product is an additive for animal or human food. In some instances, the food product is a nutritional supplement. In some instances, the food product is an animal feed. In some instances, the animal feed is an aquaculture feed. In some instances, the animal feed is a domestic animal feed, a zoological animal feed, a work animal feed, a livestock feed, or a combination thereof.

### BRIEF DESCRIPTION OF THE DRAWINGS

Figure 1 is a map of an 18S rDNA gene locus showing the origin of the fragments whose sequences are provided for characterization of the disclosed strains.
Figure 2 depicts growth characteristics of the cytostat culture of gamma irradiated WH-05554 in the presence of the lipid biosynthesis inhibitor cerulenin.
Figures 3A-C depicts the fold change in lipid over the course of the 23 hour culture period in small scale batch cultures of WH-05554 and derived strains that were selected in a cytostat that included a lipid biosynthesis inhibitor.
Figure 4 is a diagram depicting the composition of total fatty acids in progenitor strain WH-05554 and classically improved strains NH-05783, NH-06161, and NH-06181. The FAME analysis from small scale fermentation cultures shows that improved strain NH-05783 had increased myristic acid and DHA and UV treated strains NH-06161 and NH-06181 derived from improved strain NH-05783 demonstrated further increases in myristic acid and DHA.

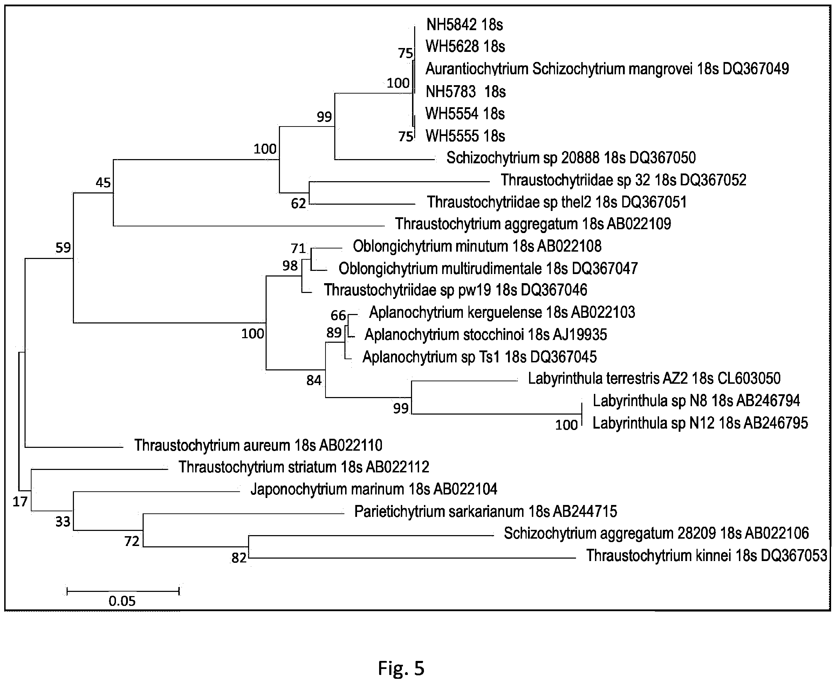

Figure 5 is a tree diagram of the relatedness of various chytrid strains placing strains WH-05628 and WH-05554 in a grouping with an *Aurantionchytrium* species.

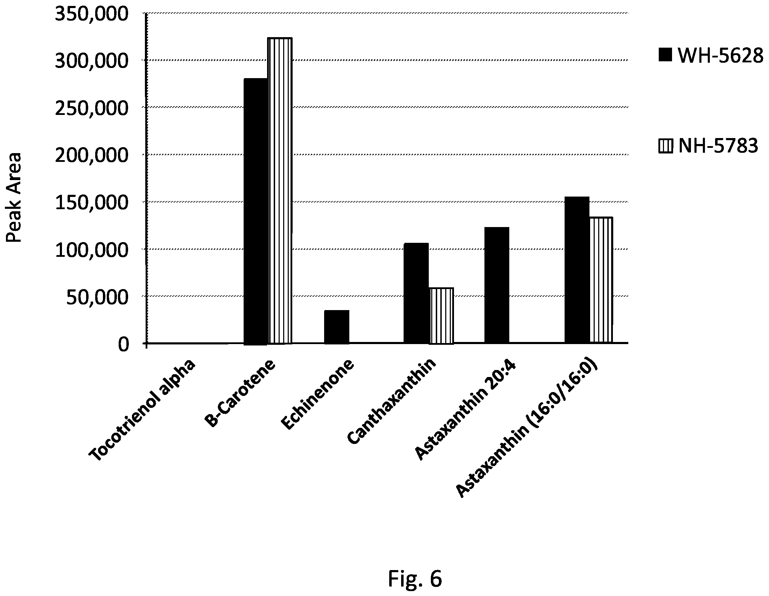

Figure 6 is a bar graph depicting the amounts of various carotenoids produced by wild type strain WH-05628 (dark bars) and classically improved strain NH-05783 (light bars).

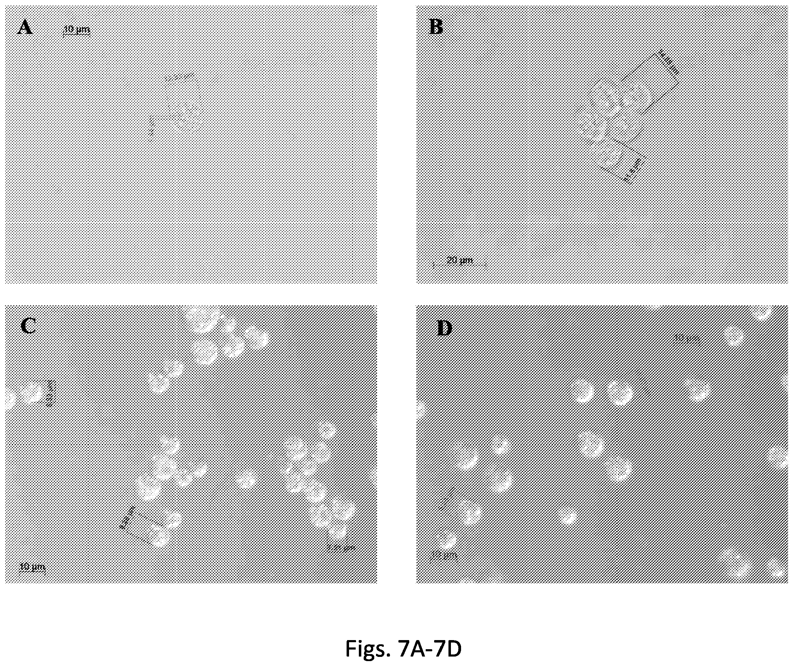

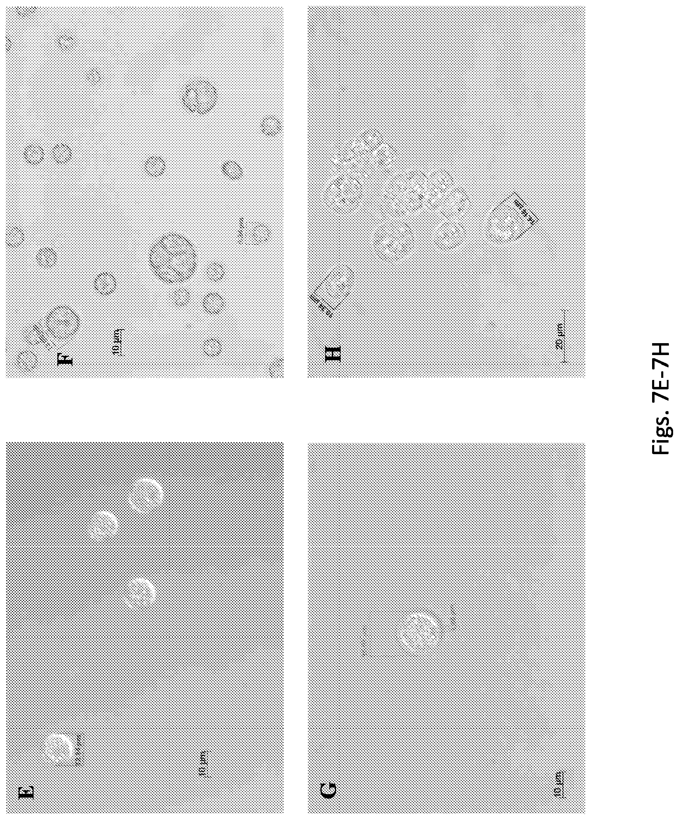

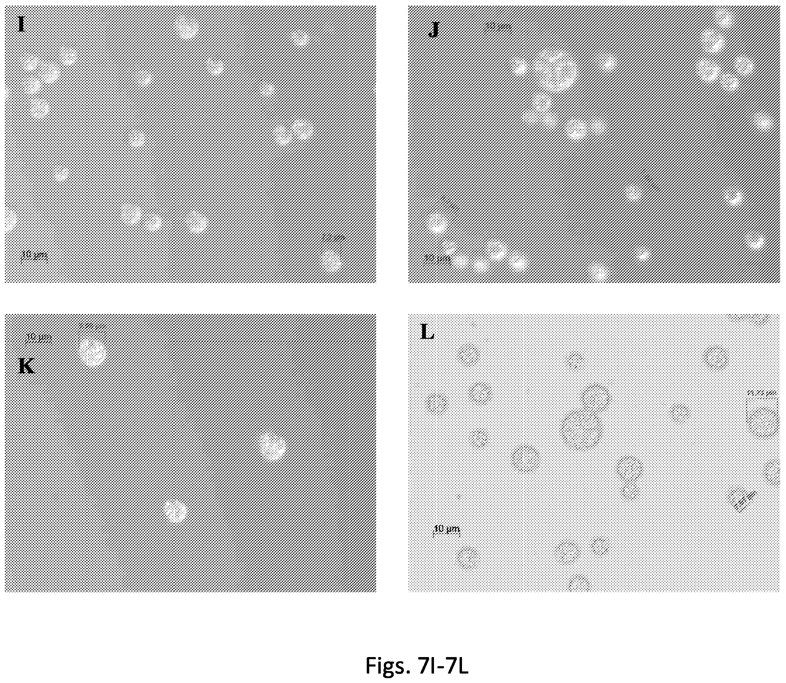

Figures 7A-L is a set of photographs from microscopy showing the morphology of cells of the Labyrinthulomycete strains of the disclosure. Classically improved strain NH-05783 is shown at A) T=0 hours, B) T=6 hours, C) T=24 hours, D) T=30 hours, E) T=48 hours, and F) T=72 hours. Isolated wild type strain WH-05628 is shown at G) T=0 hours, H) T=6 hours, I) T=24 hours, J) T=30 hours, K) T=48 hours, and L) T=72 hours.

### DETAILED DESCRIPTION

The following disclosure is only partly related to the present invention.

The invention is solely as defined in the claims.

Unless otherwise defined, all terms of art, notations and other scientific terms or terminology used herein are intended to have the meanings commonly understood by those of skill in the art.

Many of the techniques and procedures described or referenced herein are well understood and commonly employed using conventional methodology by those skilled in the art.

The singular form "a", "an", and "the" include plural references unless the context clearly dictates otherwise. For example, the term "a cell" includes one or more cells, including mixtures thereof. "A and/or B" is used herein to include all of the following alternatives: "A", "B", "A or B", and "A and B".

"About" means either: within plus or minus 10% of the provided value, or a value rounded to the nearest significant figure, in all cases inclusive of the provided value. Where ranges are provided, they are inclusive of the boundary values.

### Labyrinthulomycete Strains

Isolated strains of the labyrinthulomycete class, referred to herein as "labyrinthulomycetes" having the ability to produce polyunsaturated fatty acids (PUFAs), in particular omega-3 fatty acids such as docosahexaenoic acid (C22:6n3; DHA) are provided herein. The disclosed eukaryotic microorganisms were identified in screens designed to distinguish strains having high rates of DHA production. The strains provided herein were deposited with the Agricultural Research Service (ARS) Culture Collection located at 1815 N. University Street, Peoria, IL 61604, USA (NRRL) on April 4, 2013 by Synthetic Genomics Inc. in accordance with the Budapest Treaty. Accession numbers for these deposits are: NRRL- 50836 (strain NH-05783) and NRRL- 50837 (strain NH-06161).

**Table 1: Microbial isolates and corresponding accession numbers**

| **Strain ID** | **Accession Number** | **Taxonomy** |
|---|---|---|
| NH-SGI-05783 | NRRL-50836 | *Aurantiochytrium* sp. |
| NH-SGI-06161 | NRRL-50837 | *Aurantiochytrium* sp. |

The labyrinthulomycete is a class belonging to the Stramenopiles kingdom and includes two families, the Thraustochytriaceae and the Labyrinthulaceae Genera within the Labyrinthulomycete include *Aplanochytrium, Aurantiochytrium, Diplophrys, Japonochytrium, Labyrinthula,* Labyrinthuloides, *Oblongichytrium, Schizochytrium, Thraustochytrium,* and *Ulkenia.*

In some examples, microorganisms of the disclosure have all of the identifying characteristics of the deposited strains and, in particular, the identifying characteristics of being able to produce DHA. Particular microorganisms of the disclosure may refer to the deposited microorganisms as described above, as well as strains derived therefrom. For example, provided herein are microorganisms that are derivatives of strains deposited in the ARS culture collection under NRRL Accession No. 50836 and NRRL Accession No. 50837, in which the derivatives produce DHA. The strains may produce at least 25%, at least 30%, at least 35%, at least 40%, at least 45%, or at least 50% of their total fatty acids as DHA. The strains may additionally produce at least 6%, at least 8%, at least 10%, at least 12%, at least 15%, or at least 20% of their total fatty acids as myristic acid.

The term "lipid", as used herein, refers to fats or oils and includes free fatty acids, fatty acid derivatives such as fatty alcohols and wax esters, terpenoids, hydrocarbons (e.g., alkanes and alkenes), sterols, and glyceride esters of fatty acids, including membrane lipids or storage lipids, e.g. phospholipids, galactolipids, sulfolipids, triacylglycerols, diacylglycerols, and monoacylglycerols. Lipids that include fatty acid components (e.g., comprise acyl chains that are derived from fatty acid biosynthesis) include, for example, phospholipids, glycolipids, galactolipids, sulfolipids, triacylglycerols, diacylglycerols, and monoacylglycerols, sphingomyelin and glycosphingolipids, eicosanoids, prostaglandins, thromboxanes, leukotrienes, resolvins, protectins, isoprostanes, oxylipins. The term "total fatty acids" as used herein includes the fatty acids (linear acyl moieties) that are components of these and other cellular lipids that can be derivatized to fatty acid methyl esters (FAME) for analysis and quantitation as known in the art and described herein. Thus, "percent fatty acids", "% fatty acids" or "% total fatty acids" and "percent FAME" or "% FAME" may be used interchangeably herein when referring to the fatty acid composition of lipids or oils.

Polyunsaturated fatty acids ("PUFAs") include omega-3 and omega-6 fatty acids. Omega-3 fatty acids include, without limitation, docosahexaenoic acid (C22:6n3; DHA) and eicosapentaenoic acid (C20:5n3) EPA. Omega-6 fatty acids include, without limitation, arachidonic acid (C20:4n6; ARA) and docosapentaenoic acid (C22:5n6; DPA), gamma-linoleic acid (C18:3 n-6), eicosadienoic acid (C20:2 n-6), and eicosatrienoic acid (C20:3 n-6). Omega-3 fatty acids can also include eicosatrienoic acid (C20:3n3), stearidonic acid (C18:4n3), eicosatetraenoic acid (C20:4 n-3), octadecapentaenoic acid (C18:5 n-3).

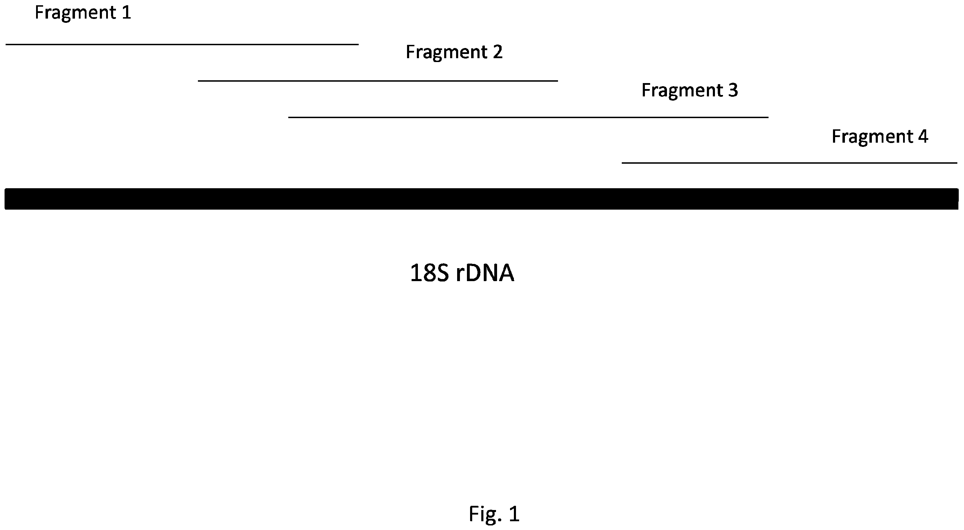

Four overlapping fragments of the 18S rRNA gene that provide unique sequences for 18S gene regions were obtained for wild type isolate WH-05554 (SEQ ID NO:1, SEQ ID NO:2, SEQ ID NO:3, and SEQ ID NO:4), as well as for derived strains NH-05783 (SEQ ID NO:5, SEQ ID NO:6, SEQ ID NO:7, and SEQ ID NO:8), and NH-06161 (SEQ ID NO:9, SEQ ID NO:10, SEQ ID NO:11, and SEQ ID NO:12). Because the 18S rRNA genes are multicopy, four fragment sequences determined from distinct sequence runs are provided separately to avoid presenting a chimeric sequence including sequences originating from different loci. A diagram mapping the fragments to an 18S rDNA locus of strain WH-05783 is provided in **Figure 1** for illustrative purposes.

As used herein, unless otherwise specified, reference to a percent (%) identity refers to an evaluation of homology which is performed using: (1) a BLAST 2.0 Basic BLAST homology search using blastp for amino acid searches and blastn for nucleic acid searches with standard default parameters, wherein the query sequence is filtered for low complexity regions by default (described in Altschul, S. F., Madden, T. L., Schaaffer, A. A., Zhang, J., Zhang, Z., Miller, W. & Lipman, D. J. (1997) "Gapped BLAST and PSI-BLAST: a new generation of protein database search programs." Nucleic Acids Res. 25:3389-3402); (2) a BLAST 2 alignment (using the parameters described below); (3) and/or PSI-BLAST with the standard default parameters (Position-Specific Iterated BLAST. It is noted that due to some differences in the standard parameters between BLAST 2.0 Basic BLAST and BLAST 2, two specific sequences might be recognized as having significant homology using the BLAST 2 program, whereas a search performed in BLAST 2.0 Basic BLAST using one of the sequences as the query sequence may not identify the second sequence in the top matches. In addition, PSI-BLAST provides an automated, easy-to-use version of a "profile" search, which is a sensitive way to look for sequence homologues. The program first performs a gapped BLAST database search. The PSI-BLAST program uses the information from any significant alignments returned to construct a position-specific score matrix, which replaces the query sequence for the next round of database searching. Therefore, it is to be understood that percent identity can be determined by using any one of these programs.

For example, two specific sequences can be aligned to one another using BLAST 2 sequence as described in Tatusova and Madden, (1999), "Blast 2 sequences--a new tool for comparing protein and nucleotide sequences", FEMS Microbiol Lett. 174:247-250. BLAST 2 sequence alignment is performed in blastp or blastn using the BLAST 2.0 algorithm to perform a Gapped BLAST search (BLAST 2.0) between the two sequences allowing for the introduction of gaps (deletions and insertions) in the resulting alignment. For purposes of clarity herein, a BLAST 2 sequence alignment is performed using the standard default parameters as follows: For blastn, using 0 BLOSUM62 matrix: Reward for match=1; Penalty for mismatch=-2 ; Open gap (5) and extension gap (2) penalties; gap x_dropoff (50); expect (10) word size (11) filter (on). For blastp, using 0 BLOSUM62 matrix: Open gap (11) and extension gap (1) penalties; gap x_dropoff(50) expect (10) word size (3) filter (on).

The fragments whose sequences are provided herein extend along the same region of the 18S rRNA gene, but begin and end at different base positions with respect to the 18S rRNA gene sequence. For example, SEQ ID NOs 1, 5, and 9 all correspond to "Fragment 1" of Figure 1 but are of different length, and may begin and/or end at different positions; SEQ ID NOs 2, 6, and 10 all correspond to "Fragment 2" of Figure 1 but may be of different length, beginning and ending at different positions; SEQ ID NOs 3, 7, and 11 all correspond to "Fragment 3" of Figure 1 but may be of different length, and may begin and/or end at different positions; and SEQ ID NOs 4, 8, and 12 all correspond to "Fragment 4" of Figure 1 but may be of different length, beginning and ending at different positions. Thus the % identity between these sequences corresponding to the same fragment of the 18S rRNA gene, may be calculated based on where the BLAST alignment begins (or ends), which may be within 10, 20, 50, 75, 100 oe 200 nucleotides of the first nucleotide of one sequence, and may be within 10, 20, 50, 75, 100 oe 200 nucleotides of the first nucleotide of one sequence. Similarly, when assessing the % identity of other sequences with respect to SEQ ID NOs: 1-12 as provided herein, the % identity can begin and end where the BLAST alignment between the two fragments begins and ends. Preferably, the % identity specificed herein are over at least 200, at least 300, or at least 400 contiguous nucleotides of any of SEQ ID NOs: 1-12, and more preferably over at least 500 nucleotides of any of SEQ ID NOs: 1-12.

The disclosure provides a mutant microorganism of the heterokont labyrinthulomycete class having an 18S ribosomal RNA gene that includes a sequence that has at least 95%, 96%, 97%, 97.5%, 98%, 98.1%, 98.2%, 98.3%, 98.4%, 98.5%, 98.6%, 98.7%, 98.8%, 98.9%, 99%, 99.1%, 99.2%, 99.3%, 99.4%, 99.5%, 99.6%, 99.7%, 99.8%, or 99.9% identity to SEQ ID NO:1, SEQ ID NO:2, SEQ ID NO:3, or SEQ ID NO:4. In some examples, the mutant microorganism has an 18S ribosomal RNA gene that includes a sequence that has at least 95%, 96%, 97%, 97.5%, 98%, 98.1%, 98.2%, 98.3%, 98.4%, 98.5%, 98.6%, 98.7%, 98.8%, 98.9%, 99%, 99.1%, 99.2%, 99.3%, 99.4%, 99.5%, 99.6%, 99.7%, 99.8%, or 99.9% identity to SEQ ID NO:1, SEQ ID NO:2, SEQ ID NO:3, and SEQ ID NO:4. The microorganism can preferably produce a microbial oil in which at least 20%, at least 25%, at least 30%, at least 35%, at least 40%, about 40%, or at least 45% of the fatty acids of the produced oil are DHA, and can preferably produce a microbial oil in which at least 20%, at least 25%, at least 30%, at least 35%, at least 40%, about 40%, or at least 45% of the fatty acids of the produced microbial oil are DHA in the absence of a lipid biosynthesis inhibitor. The mutant microorganism can additionally produce, *e.g.,* a microbial oil, in which at least 6%, at least 8%, at least 10%, at least 12%, at least 15%, at least 20%, at least 25%, or at least 30% of the fatty acids are myristic acid, and can preferably produce lipid in which at least 20%, at least 25%, at least 30%, at least 35%, at least 40%, about 40%, or at least 45% of the fatty acids of the produced microbial oil are DHA in the absence of a lipid biosynthesis inhibitor. In some examples, the microorganism produces a microbial oil in which less than 50%, less than 40%, less than 30%, or less than 20% of the fatty acids are palmitic acid. For example, the mutant microorganism may produce a higher percentage of its total fatty acids as myristic acid than as palmitic acid. In some examples, the mutant microorganism can produce DHA at a rate of at least 45 mg/liter/hour in small scale batch cultures. In some examples, the strain can preferably produce DHA at a rate of at least 45 mg/L/h, about 50 mg/L/h, at least 50 mg/L/h, at least 100 mg/L/h, at least 130 mg/L/h, at least 160 mg/L/h, at least 190 mg/L/h, or at least 200mg/L/h in batch cultures ranging from 2-10 ml in volume, for example, from 4-8 ml in volume, such as from 5-6 ml in volume, that are incubated from about 12 to about 24 hours. In preferred examples, the strain can produce lipid in which the ratio of DHA to docosapentaenoic acid (DPA) is at least about 3.5:1 or at least about 4.0 to 1. The microorganism can preferably produce lipid in which less than 5%, less than 2%, less than 1%, or less than about 0.5% of the fatty acids are EPA. Additionally, the microorganism preferably produces lipid in which less than 2%, less than 1%, less than about 0.5%, or less than about 0.2% of the fatty acids are ARA.

The disclosure also provides a mutant microorganism of the heterokont labyrinthulomycete class having an 18S ribosomal RNA gene that includes a sequence that has at least 95%, 96%, 97%, 97.5%, 98%, 98.1%, 98.2%, 98.3%, 98.4%, 98.5%, 98.6%, 98.7%, 98.8%, 98.9%, 99%, 99.1%, 99.2%, 99.3%, 99.4%, 99.5%, 99.6%, 99.7%, 99.8%, or 99.9% identity to SEQ ID NO:5, SEQ ID NO:6, SEQ ID NO:7, or SEQ ID NO:8. In some examples, the mutant microorganism has an 18S ribosomal RNA gene that includes a sequence that has at least 95%, 96%, 97%, 97.5%, 98%, 98.1%, 98.2%, 98.3%, 98.4%, 98.5%, 98.6%, 98.7%, 98.8%, 98.9%, 99%, 99.1%, 99.2%, 99.3%, 99.4%, 99.5%, 99.6%, 99.7%, 99.8%, or 99.9% identity to SEQ ID NO:5, SEQ ID NO:6, SEQ ID NO:7, and SEQ ID NO:8. The microorganism can preferably produce a microbial oil in which at least 20%, at least 25%, at least 30%, at least 35%, at least 40%, about 40%, or at least 45% of the fatty acids of the produced oil are DHA, and can preferably produce a microbial oil in which at least 20%, at least 25%, at least 30%, at least 35%, at least 40%, about 40%, or at least 45% of the fatty acids of the produced microbial oil are DHA in the absence of a lipid biosynthesis inhibitor. The mutant microorganism can additionally produce, *e.g.,* a microbial oil, in which at least 6%, at least 8%, at least 10%, at least 12%, at least 15%, at least 20%, at least 25%, or at least 30% of the fatty acids are myristic acid, and can preferably produce lipid in which at least 20%, at least 25%, at least 30%, at least 35%, at least 40%, about 40%, or at least 45% of the fatty acids of the produced microbial oil are DHA in the absence of a lipid biosynthesis inhibitor. In some examples, the microorganism produces a microbial oil in which less than 50%, less than 40%, less than 30%, or less than 20% of the fatty acids are palmitic acid. For example, the mutant microorganism may produce a higher percentage of its total fatty acids as myristic acid than as palmitic acid. In some examples, the mutant microorganism can produce DHA at a rate of at least 45 mg/liter/hour in small scale batch cultures. In some examples, the strain can preferably produce DHA at a rate of at least 45 mg/L/h, about 50 mg/L/h, at least 50 mg/L/h, at least 100 mg/L/h, at least 130 mg/L/h, at least 160 mg/L/h, at least 190 mg/L/h, or at least 200 mg/L/h in batch cultures ranging from 2-10 ml in volume, for example, from 4-8 ml in volume, such as from 5-6 ml in volume, that are incubated from about 12 to about 24 hours. In preferred examples, the strain can produce lipid in which the ratio of DHA to docosapentaenoic acid (DPA) is at least about 3.5:1 or at least about 4.0 to 1. The microorganism can preferably produce lipid in which less than 5%, less than 2%, less than 1%, or less than about 0.5% of the fatty acids are EPA. Additionally, the microorganism preferably produces lipid in which less than 2%, less than 1%, less than about 0.5%, or less than about 0.2% of the fatty acids are ARA. In some examples, the mutant microorganism is NH-05783 (NRRL-50836) or a strain derived therefrom.

The disclosure also provides a mutant microorganism of the heterokont labyrinthulomycete class having an 18S ribosomal RNA gene that includes a sequence that has at least 95%, 96%, 97%, 97.5%, 98%, 98.1%, 98.2%, 98.3%, 98.4%, 98.5%, 98.6%, 98.7%, 98.8%, 98.9%, 99%, 99.1%, 99.2%, 99.3%, 99.4%, 99.5%, 99.6%, 99.7%, 99.8%, or 99.9% identity to SEQ ID NO:9, SEQ ID NO:10, SEQ ID NO:11, or SEQ ID NO:12. In some examples, the mutant microorganism has an 18S ribosomal RNA gene that includes a sequence that has at least 95%, 96%, 97%, 97.5%, 98%, 98.1%, 98.2%, 98.3%, 98.4%, 98.5%, 98.6%, 98.7%, 98.8%, 98.9%, 99%, 99.1%, 99.2%, 99.3%, 99.4%, 99.5%, 99.6%, 99.7%, 99.8%, or 99.9% identity to SEQ ID NO:9, SEQ ID NO:10, SEQ ID NO:11, and SEQ ID NO:12. The microorganism can preferably produce a microbial oil in which at least 20%, at least 25%, at least 30%, at least 35%, at least 40%, about 40%, or at least 45% of the fatty acids of the produced oil are DHA, and can preferably produce a microbial oil in which at least 20%, at least 25%, at least 30%, at least 35%, at least 40%, about 40%, or at least 45% of the fatty acids of the produced microbial oil are DHA in the absence of a lipid biosynthesis inhibitor. The mutant microorganism can additionally produce, *e.g.,* a microbial oil, in which at least 6%, at least 8%, at least 10%, at least 12%, at least 15%, at least 20%, at least 25%, or at least 30% of the fatty acids are myristic acid, and can preferably produce lipid in which at least 20%, at least 25%, at least 30%, at least 35%, at least 40%, about 40%, or at least 45% of the fatty acids of the produced microbial oil are DHA in the absence of a lipid biosynthesis inhibitor. In some examples, the microorganism produces a microbial oil in which less than 50%, less than 40%, less than 30%, or less than 20% of the fatty acids are palmitic acid. For example, the mutant microorganism may produce a higher percentage of its total fatty acids as myristic acid than as palmitic acid. In some examples, the mutant microorganism can produce DHA at a rate of at least 45 mg/liter/hour in small scale batch cultures. In some examples, the strain can preferably produce DHA at a rate of at least 45 mg/L/h, about 50 mg/L/h, at least 50 mg/L/h, at least 100 mg/L/h, at least 130 mg/L/h, at least 160 mg/L/h, at least 190 mg/L/h, or at least 200mg/L/h in batch cultures ranging from 2-10 ml in volume, for example, from 4-8 ml in volume, such as from 5-6 ml in volume, that are incubated from about 12 to about 24 hours. In preferred examples, the strain can produce lipid in which the ratio of DHA to docosapentaenoic acid (DPA) is at least about 3.5:1 or at least about 4.0 to 1. The microorganism can preferably produce lipid in which less than 5%, less than 2%, less than 1%, or less than about 0.5% of the fatty acids are EPA. Additionally, the microorganism preferably produces lipid in which less than 2%, less than 1%, less than about 0.5%, or less than about 0.2% of the fatty acids are ARA. In some examples, the mutant microorganism is NH-06161 (NRRL-50837) or a strain derived therefrom.

The term "derivative", when referring to a strain, encompasses mutants and variants of a strain or its descendants. Thus, for example, a derivative strain of a wild type strain can be derived directly from a wild type or native strain, or can be derived from a strain that itself was derived (directly or indirectly) from a wild type or native strain.

In various instances, the mutant strains provided herein can produce at least 50 mg/L/h, at least 100 mg/L/h, at least 150 mg/L/h, at least 200 mg/L/h, at least 250 mg/L/h, at least 300 mg/L/h, at least 350 mg/L/h, or at least 400 mg/L/h DHA in cultures having volumes of at least 100 ml, at least 200 ml, at least 500 ml, or at least 1 liter.

The disclosure also provides mutant labyrinthulomycete strains in which the strains are mutants having increased myristic acid as a percentage of total fatty acids produced by the strains with respect to the wild type or progenitor strains from which they are derived. Such mutant strains can be strains of, for example, a genus such as Labyrinthula, Labyrinthuloides, *Thraustochytrium, Schizochytrium, Aplanochytrium, Aurantiochytrium, Japonochytrium, Oblongichytrium, Diplophrys,* or *Ulkenia.* For example, a mutant strain as provided herein can be a strain of *Thraustochytrium, Schizochytrium,* or *Aurantiochytrium.* For example, a mutant strain that produces a higher percentage of fatty acids as myristic acid can be an *Aurantiochytrium* or *Schizochytrium* strain having an 18S rDNA sequence at least 95%, at least 96%, at least 97%, at least 98%, at least 98.5%, at least 99%, at least 99.1%, at least 99.2%, at least 99.3%, at least 99.4%, at least 99.5%, at least 99.6%, at least 99.7%, at least 99.8%, at least 99.9%, or 100% identical to any of SEQ ID NO:1, SEQ ID NO:2, SEQ ID NO:3, SEQ ID NO:4, SEQ ID NO:5, SEQ ID NO:6, SEQ ID NO:7, SEQ ID NO:8; SEQ ID NO:9, SEQ ID NO:10, SEQ ID NO:11, and SEQ ID NO:12. The percent myristic acid as total fatty acids produced can be increased by 50% or more in a mutant labyrinthulomycete microorganism when compared with the percent myristic acid produced by the strain from which it was derived. In various examples, the percent myristic acid as total fatty acids produced is increased by at least 100% in a mutant as compared to the strain from which it is derived.

In various examples, a mutant labyrinthulomycete strain as provided herein that produces a higher percentage of total fatty acids as myristic acid than is produced by the strain from which it is derived can produce fatty acids in which at least 10%, at least 20%, or at least 30% of the total fatty acids are myristic acid. The strain may be resistant to triclosan and/or cerulenin. The microorganism can preferably produce lipid in which at least 10%, at least 20%, at least 25%,or at least 30%, of the produced fatty acids are myristic acid in the absence of a lipid biosynthesis inhibitor in the culture. The strains may additionally produce more of a PUFA as a percent of total fatty acids.

For example, the disclosure also provides mutant labyrinthulomycete strains in which the strains are mutants having increased DHA as a percentage of total fatty acids produced and additionally have increased myristic acid as a percentage of total fatty acids produced, with respect to the wild type strains from which they are derived. For example, the percent DHA as total fatty acids produced can be increased by 20% or more and the percent myristic acid as total fatty acids produced can be increased by 50% or more in a mutant labyrinthulomycete microorganism when compared with the percent DHA and percent myristic acid produced by the strain from which it was derived. In various examples, the percent DHA of the total fatty acids produced is increased by at least 30% and the percent myristic acid as total fatty acids produced is increased by at least 100% in a mutant as compared to the strain from which it is derived. The strains may be resistant to a fatty acid synthase inhibitor such as cerulenin and/or triclosan.

In various examples, a mutant labyrinthulomycete strain as provided herein that produces a higher percentage of total fatty acids as docosahexaenoic acid (DHA) and a higher percentage of total fatty acids as myristic acid than is produced by the strain from which it is derived can produce fatty acids in which at least 25%, at least 30%, at least 35%, or at least 39% of the total fatty acids are DHA and at least 10%, at least 20%, or at least 30% of the total fatty acids are myristic acid. A mutant labyrinthulomycete strain as provided herein that produces a higher percentage of total fatty acids as docosahexaenoic acid (DHA) and a higher percentage of total fatty acids as myristic acid than is produced by the strain from which it is derived can produce fatty acids in which at least 25%, at least 30%, at least 35%, or at least 39% of the total fatty acids are DHA and at least 10%, at least 20%, or at least 30% of the total fatty acids are myristic acid in the absence of a lipid biosynthesis inhibitor in the culture. A mutant labyrinthulomycete strain as provided herein can produce DHA in small scale batch fermentation culture at a rate of at least 150 mg/L/h, at least 160 mg/L/h, at least 170 mg/L/h, at least 180 mg/L/h, at least 190 mg/L/h, at least 200 mg/L/h, or at least 210 mg/L/h. A mutant labyrinthulomycete strain as provided herein can produce DHA at a rate higher than that of the strain from which it is derived.

The total fatty acids produced by a mutant labyrinthulomycete strain can include, for example, 10% or less of DPA, 9% or less of DPA, 8% or less of DPA, 7% or less of DPA, 10% or less of DPA, or 6% or less of DPA. The ratio of DHA to DPA in fatty acids produced by a mutant labyrinthulomycete strain as provided herein can be about 4 : 1 or higher, for example, about 4.5 : 1 or higher, about 4.9 : 1 or higher, or about 5 : 1.

The disclosure also provides an isolated labyrinthulomycete biomass comprising a mutant labyrinthulomycete microorganism as provided herein. In some instances, at least 20%, at least 30%, at least 40%, at least 50%, at least 60%, at least 70%, or at least 80% of the dry cell weight of the biomass are fatty acids. In some instances, the biomass comprises at least 20%, at least 30%, at least 40%, at least 50%, at least 60%, at least 70%, or at least 80% by weight of the fatty acids as DHA. In some instances, the biomass comprises at least 6%, at least 8%, at least 10%, at least 12%, at least 15%, at least 20%, at least 25%, or at least 30% by weight of the fatty acids as myristic acid.In some instances, the biomass comprises 6% or less, 5% or less, 4% or less, 3% or less, 2% or less, 1% or less, or 0.55% or less, by weight of the fatty acids as EPA. In some instances, the biomass is substantially free of EPA. In some instances, the biomass comprises from about 0.5% to about 1%, about 0.5% to about 2%, about 0.5% to about 5%, about 0.5% to about 6%, about 1% to about 5%, about 1% to about 6%, about 2% to about 5%, or about 2% to about 6%, or about 2% to about 10%, by weight of the fatty acids as DPA.

### Microbial Oil

The disclosure is further directed to a microbial oil produced by a microorganism provided herein. Although a microbial oil of the disclosure can be any oil derived from a microorganism, including, for example: a crude oil extracted from the biomass of the microorganism without further processing; a refined oil that is obtained by treating a crude microbial oil with further processing steps such as refining, bleaching, and/or deodorizing; a diluted microbial oil obtained by diluting a crude or refined microbial oil; or an enriched oil that is obtained, for example, by treating a crude or refined microbial oil with further methods of purification to increase the concentration of a fatty acid (such as DHA) in the oil, unless otherwise specified, a microbial oil as disclosed herein is a crude oil extracted from an organism or biomass of the organism without further processing. For example, a crude oil may be obtained for example by extraction of lysed or unlysed cells with solvents, such as but not limited to organic or water miscible solvents including hydrocarbons such as hexane, alcohols such as methanol, chloroform, methylene chloride, etc.

The fatty acid content of a crude oil as disclosed herein is commonly determined by fatty acid methyl ester (FAME) analysis as described in the Examples herein. As used herein, "fatty acids" (as well as other fatty acid terms such as "omega-3 fatty acid", "PUFA", "DHA", "myristic acid", and the like), when referring to the fatty acid content of cellular lipid or a microbial oil, refers to, in addition to free fatty acids, fatty acid in the context of lipids (*e.g.*, as acyl chains esterified or otherwise biochemically conjugated to other chemical moieties) such as but not limited to triglycerides, diglycerides, and monoglycerides, wax esters, and polar lipids such as phospholipids. Thus, the percentage of a particular fatty acid species such as DHA in the total fatty acids of a microbial oil includes the fatty acid present in, for example, glycerolipids and phospholipids and is expressed as a percentage of total fatty acids or "percent FAME lipid".

In some instances, the microbial oil comprises at least 20%, at least 25%, at least 30%, at least 35%, at least 40%, at least 45%, at least 50%, at least 55%, at least 60%, at least 65%, at least 70%, at least 75%, or at least 80% by weight DHA. In some examples the microbial oil can contain at least 10%, at least 20%, or at least 30% of the fatty acids of the lipid as myristic acid. In some instances, the microbial oil comprises about 10% or less, about 9% or less, about 8% or less, about 7% or less, about 6% or less, about 5% or less, about 4% or less, about 3% or less, about 2% or less, or about 1% or less of FAME as EPA. In some instances, the microbial oil is substantially free of EPA. In some instances, the microbial oil comprises from about 0.5% to about 1%, about 0.5% to about 2%, about 0.5% to about 2.5%, about 0.5% to about 3%, about 0.5% to about 3.5%, about 0.5% to about 5%, about 0.5% to about 6%, about 1% to about 2%, about 2% to about 3%, about 2% to about 3.5%, about 1% to about 2.5%, about 1% to about 3%, about 1% to about 3.5%, about 1% to about 5%, or about 1% to about 6% of FAME as DPAn6. In some instances, the microbial oil comprises a weight ratio of DHA to DPAn6 of equal to or greater than about 4:1, of at least 5:1, or of at least 6:1.

### Lipid Production

Lipids can be produced using one or more isolated Labyrinthulomycete microoganisms of the disclosure or a derivative thereof. Various fermentation parameters for inoculating, growing, and recovering biomass from labyrinthulomycetes are known in the art, such as described in U.S. Pat. No. 5,130,242; US 6.582,941; US, 8,207,363; US 6,607,900, US 6,607,900; and US Patent Application Publication US20080155705.

Any medium for growth of labyrinthulomycete microorganisms can be used. For example, recipes for cultivating labyrinthulomycetes can be found in US Patent 8,207,363, and are also provided in the Examples herein. The culture medium can optionally contain natural or artificial sea water that can be present at a dilution of, for example 1% to 99% of the final media formulation. A culture medium for labyrinthulomycete microorganisms includes at least one carbon source for the microorganism. Examples of carbon sources that can be present in the culture medium include, but are not limited to, glucose, fructose, galactose, L-fucose (derived from galactose), lactose, lactulose, maltose, maltriose xylose, saccharose, soluble starch, dextrin (derived from corn) and alpha-cyclodextrin (derived from starch), glycogen, gelatin, molasses, corn steep liquor, m-inositol (derived from corn steep liquor), glucosamine, dextran, fats, oils, glycerol, acetate (*e.g*., sodium acetate, potassium acetate), acetic acid, mannitol, ethanol, galacturonic acid (derived from pectin), cellobiose (derived from cellulose) and polyols such as maltitol, erythritol, adonitol and oleic acids such as glycerol and tween 80 and amino sugars such as N-acetyl-D-galactosamine, N-acetyl-D-glucosamine and N-acetyl-β-D-mannosamine. The culture medium can include a nitrogen source, which can be, for example, an inorganic nitrogen source, such as ammonium acetate, ammonium sulfate, ammonium chloride, or ammonium nitrate. Alternatively or in addition, a nitrogen source provided in the culture medium can be an organic nitrogen source, including, as nonlimiting examples, peptone, yeast extract, polypeptone, malt extract, soy flour, meat extract, fish meal, casamino acids, corn steep liquor, glutamate, or urea. The culture medium also includes a form of phosphate, such as potassium phosphate or sodium-phosphate, and inorganic-salts, acids, or bases such as, for example, ammonium sulfate, ammonium hydroxide, magnesium chloride, magnesium sulfate, potassium hydroxide, sodium bicarbonate, boric acid, citric acid, phosphoric acid, sodium orthovanadate, potassium chromate, potassium chloride, sodium chloride, sodium sulfate, sodium molybdate, selenous acid, nickel sulfate, copper sulfate, zinc sulfate, cobalt chloride, iron chloride, manganese chloride and calcium chloride that can supply nutrients, including trace nutrients. One or more chelating compounds (*e.g*., ethylenediaminetetraacetic acid, citric acid or citrate) can also be present in the culture medium. Additionally, one or more vitamins such as but not limited to pyridoxine hydrochloride, thiamine hydrochloride, calcium pantothenate, p-aminobenzoic acid, riboflavin, nicotinic acid, biotin, folic acid and vitamin B₁₂ may be present as a media component.

In some instances, the culture medium comprises at least 5%, at least 10%, at least 20%, at least 30%, at least 40%, at least 50%, at least 60%, at least 70%, at least 80%, or at least 90% dissolved oxygen, as a percentage of saturation level. In some instances, the culture medium comprises from about 5% to about 20%, about 5% to about 50%, about 5% to about 100%, about 10% to about 20%, about 10% to about 50%, about 10% to about 100%, about 20% to about 50%, or about 20% to about 100% dissolved oxygen, as a percentage of saturation level.

The fermentation volume can be any feasible volume. In some instances, the fermentation volume (volume of culture) is at least about 1 liter or at least about 2 liters, at least about 5 liters, at least about 10 liters, at least about 50 liters, at least about 100 liters, at least about 200 liters, at least about 500 liters, at least about 1000 liters, at least about 10,000 liters, at least about 20,000 liters, at least about 50,000 liters, at least about 100,000 liters, at least about 150,000 liters, at least about 200,000 liters, or at least about 250,000 liters. In some instances, the fermentation volume is about 1 liter to about 300,000 liters, about 2 liters, about 10 liters, about 50 liters, about 100 liters, about 200 liters, about 500 liters, about 1000 liters, about 10,000 liters, about 20,000 liters, about 50,000 liters, about 100,000 liters, about 150,000 liters, about 200,000 liters, about 250,000 liters, or about 300,000 liters.

Fermentation can be conducted at a temperature of from about 15°C to about 40°C, for example from about 17°C to about 35°C, or from about 18°C to about 35°C, or from about 20°C to about 32°C, or from about 22°C to about 30°C. For example, at least one stage of fermentation can be performed at a temperature of about 17°C, about 18°C, about 19°C, about 20°C, about 21°C, about 22°C, about 23°C, about 24°C, about 25°C, about 26°C, about 27°C, about 28°C, about 29° C, about 30°C, about 31°C, about 32°C, about 33°C, about 34°C, or about 35°C. The culture medium can have a pH of from about 4.0 to about 8.5, for example, the culture medium can have a pH of from about 4.2 to about 8.0, or from about pH 4.5 to about pH 7.8, or from about 5.0 to about 7.5, for example fermentation can be in a medium at from about pH 4.5 to about pH 5.0, from about pH 5.0 to about pH 5.5, from about pH 5.5 to about pH 6.0,from about pH 6.0 to about pH 6.5, from about pH 6.5 to about pH 7.0, from about pH 7.0 to about pH 7.5, from about pH 7.5 to about pH 8.0, or from about pH 8.0 to about pH 8.5.

Cultivation can be carried out for 1 to 30 days, 1 to 21 days, 1 to 15 days, 1 to 12 days, 1 to 9 days, 1 to 7 days, or 1 to 5 days at temperatures between 4 to 40°C, preferably 18 to 35°C, by aeration-shaking culture, shaking culture, stationary culture, batch culture, fed-batch culture, continuous culture, rolling batch culture, or wave culture, or the like. In various culture methods, there may be two or more culture phases (for example, a growth phase and a lipid production phase) that may differ in, for example, temperature, dissolved oxygen concentration, degree of stirring or agitation, availability of one or more nutrients, etc.

In some instances, culture of an isolated labyrinthulomycete as provided herein has an omega-3 fatty acid productivity of at least about 2 g/L/day, at least about 4 g/L/day, or at least about 8 g/L/day during the cultivation period at about 15°C to about 35°C in a culture medium of about pH 4.5 to about pH 8.0 comprising sources of carbon, nitrogen, and nutrients. In some instances, the isolated labyrinthulomycete culture has an omega-3 fatty acid productivity of between about 1 g/L/day to about 30 g/L/day, about 2 g/L/day to about 25 g/L/day, about 2 g/L/day to about 25 g/L/day, about 3 g/L/day to about 20 g/L/day, or about 4 g/L/day to about 20 g/L/day during the cultivation period at about 20°C to about 35°C in a culture medium of about pH 4.5 to about pH 7.5 comprising sources of carbon, nitrogen, and other nutrients.

### Extraction

A variety of procedures can be employed in the recovery of the resultant cellular biomass from fermentation in various culture media, such as by filtration or centrifugation. The cells can then be washed, frozen, lyophilized, or spray dried, and stored under a non-oxidizing atmosphere to eliminate the presence of oxygen, prior to incorporation into a processed food or feed product.

The lipid containing DHA can be obtained by breaking or disrupting the collected cell biomass, for example, via milling, ultrasonication, or any other convenient means, and then carrying out extraction with a solvent such as chloroform, hexane, methylene chloride, methanol, ethanol or via supercritical fluid extraction means. The omega-3 polyunsaturated fatty acids may be further concentrated by hydrolyzing the lipids and concentrating the highly unsaturated fraction by employing traditional methods such as urea adduction or fractional distillation, column chromatography, or by supercritical fluid fractionation. The cells can also be broken or lysed and the lipids extracted into vegetable or animal (*e.g*. fish oils) oils. The extracted oils can be refined by well-known processes routinely employed to refine vegetable oils (*e.g.* by chemical or physical refining). These refining processes remove impurities from extracted oils before they are used or sold as edible oils. After refining, the oils can be used directly as a feed or food additive to produce omega-3 and/or omega-6 enriched products. Alternatively, the oil can be further processed and purified as outlined below and then used in the above applications and also in pharmaceutical applications.

In another process for the production of enriched (concentrated) omega-3 or omega-6 oils, the harvested cellular biomass (fresh or dried) can be ruptured or permeabilized by well-known techniques such as sonication, liquid-shear disruption methods, bead milling, pressing under high pressure, freeze-thawing, or enzymatic digestion of the cell wall. The lipids from the ruptured cells are extracted by use of a solvent or mixture of solvents such as hexane, chloroform, ether, or methanol. The solvent is removed and the lipids hydrolyzed by using any of the well-known methods for converting triglycerides to free fatty acids or esters of fatty acids including base, acid, or enzymatic hydrolysis. After hydrolysis is completed, the nonsaponifiable compounds are extracted into a solvent such as ether, hexane or chloroform and removed. The remaining solution is then acidified by addition of an acid, and the free fatty acid extracted into a solvent such as hexane, ether or chloroform. The solvent solution containing the free fatty acids can then be cooled to a temperature low enough for crystallization of the non-PUFA compounds, which can then be removed via filtration, centrifugation or settling. Resulting in the concentration of the remaining PUFA compounds and used as a nutritional supplements for humans, as a food additive, or as pharmaceutical applications.

### Products

Compositions of the disclosure include, but are not limited to, food products, pharmaceutical compositions, cosmetics, and industrial compositions.

A food product that may include a microbial oil as provided herein includes both solid and liquid compositions. A food product can be an additive to animal or human foods. Foods include, but are not limited to, common foods; liquid products, including milks, beverages, therapeutic drinks, and nutritional drinks; functional foods; supplements; nutraceuticals; infant formulas, including formulas for pre-mature infants; foods for pregnant or nursing women; foods for adults; geriatric foods; and animal foods.

A labyrinthulomycete biomass or microbial oil can be used directly as or included as an additive within one or more of: an oil, shortening, spread, other fatty ingredient, beverage, sauce, dairy-based or soy-based food (such as milk, yogurt, cheese and ice-cream), a baked good, a nutritional product, e.g., as a nutritional supplement (in capsule or tablet form), a vitamin supplement, a diet supplement, a powdered drink, a finished or semi-finished powdered food product, and combinations thereof.

In some instances, the composition is an animal feed, including without limitation, feed for aquatic animals and terrestrial animals. In some instances, the composition is a feed or feed supplement for any animal whose meat or products are consumed by humans, such as any animal from which meat, eggs, or milk is derived for human consumption. When fed to such animals, nutrients such as LC-PUFAs can be incorporated into the flesh, milk, eggs or other products of such animals to increase their content of these nutrients.

In some instances, the composition is a pharmaceutical composition. Suitable pharmaceutical compositions include, but are not limited to, an anti-inflammatory composition, a drug for treatment of coronary heart disease, a drug for treatment of arteriosclerosis, a chemotherapeutic agent, an active excipient, an osteoporosis drug, an antidepressant, an anti-convulsant, an anti-Helicobacter pylori drug, a drug for treatment of neurodegenerative disease, a drug for treatment of degenerative liver disease, an antibiotic, a cholesterol lowering composition, and a triglyceride lowering composition. In some instances, the composition is a medical food. A medical food includes a food that is in a composition to be consumed or administered externally under the supervision of a physician and that is intended for the specific dietary management of a condition, for which distinctive nutritional requirements, based on recognized scientific principles, are established by medical evaluation.

The microbial oil can be formulated in a dosage form. Dosage forms can include, but are not limited to, tablets, capsules, cachets, pellets, pills, powders and granules, and parenteral dosage forms, which include, but are not limited to, solutions, suspensions, emulsions, and dry powders comprising an effective amount of the microbial oil. It is also known in the art that such formulations can also contain pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like. Administration forms can include, but are not limited to, tablets, dragees, capsules, caplets, and pills, which contain the microbial oil and one or more suitable pharmaceutically acceptable carriers.

For oral administration, the microbial oil can be combined with pharmaceutically acceptable carriers well known in the art. Such carriers enable the microbial oils of the invention to be formulated as tablets, pills, dragees, capsules, liquids, gels, syrups, slurries, suspensions and the like, for oral ingestion by a subject to be treated. In some instances, the dosage form is a tablet, pill or caplet. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragacanth, methyl cellulose, hydroxypropylmethyl cellulose, sodium carboxymethyl cellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate. Pharmaceutical preparations that can be used orally include, but are not limited to, push-fit capsules made of gelatin, as well as soft, sealed capsules made of gelatin and a plasticizer, such as glycerol or sorbitol.

In further instances, the composition is a cosmetic or a personal care product. Cosmetics and personal care products include, but are not limited to, emulsions, creams, lotions, masks, soaps, shampoos, shaving crèmes, washes, facial creams, conditioners, make-ups, bath agents, and dispersion liquids. Cosmetic agents can be medicinal or non-medicinal.

### Methods for Isolating Strain Derivatives

Further provided herein are methods for isolating microbial strains having enhanced lipid productivity, where the methods include: culturing a microorganism of a progenitor strain in a cytostat or chemostat that includes a culture medium that includes at least one compound that inhibits the activity of an enzyme that participates in lipid metabolism to provide an inhibitor-selected population, and isolating a microorganism from the inhibitor-selected population that has improved lipid productivity with respect to the progenitor microorganism strain to provide a derivative strain having enhanced lipid productivity. Enhanced lipid productivity includes, without limitation, increased total production or production rate of any class of lipids or specific lipids, including, without limitation: total lipid, neutral lipids, FAME lipids ("FAME"), total fatty acids, any fatty acid or fatty acid derivative (e.g., one or more fatty acids, fatty alcohols, fatty acid esters, wax esters, alkanes, or alkenes), triglycerides (TAG), unsaturated fatty acids, oleic acid, omega-3 fatty acids, DHA, EPA, omega-6 fatty acids, ARA, saturated fatty acids, palmitic acid, myristic acid, etc. In some examples, the culturing is done in a cytostat to provide a cytostat-selected population. In some examples, the microorganism is subjected to a mutagenesis procedure prior to culturing in a cytostat or chemostat. In some examples, prior to culturing the microorganism in the presence of an inhibitor of an enzyme or factor that participates in lipid biosynthesis, the microorganism is cultured in a cytostat or chemostat in the absence of an inhibitor of lipid biosynthesis and one or more microorganisms having improved growth properties may be selected. In some examples, a microorganism that is subjected to a mutagenesis procedure can be treated with UV irradiation following the mutagenesis procedure. The post-mutagenesis UV treatment can occur prior to cytostat or chemostat culturing, or can occur after the mutagenized population has been selected in a cytostat or chemostat that includes an inhibitor of an enzyme or factor that participates in lipid biosynthesis, such that an inhibitor-selected population of mutagenized microorganisms is treated with UV. In some examples selection is in the presence of a lipid biosynthesis inhibitor, and can be, for example, in a cytostat or chemostat.

A microorganism or strain can be subjected to multiple mutagenesis procedures that optionally but preferably are each followed by culturing in a cytostat or chemostat that includes at least one compound that inhibits the activity of an enzyme or factor that participates in lipid metabolism. UV irradiation can optionally be performed on microorganisms after one, more than one, or all of the mutagenesis procedures, where multiple mutagenesis procedures are performed.

Thus, provided herein are methods for isolating microbial strains having enhanced lipid productivity, where the methods include: subjecting microorganisms of a progenitor strain to a mutagenesis procedure, culturing the mutagenized microorganisms in a cytostat or chemostat in the presence of an inhibitor of an enzyme or factor that participates in lipid biosynthesis to provide an inhibitor-selected mutagenized population, and isolating from the inhibitor-selected mutagenized population at least one microorganism of a derivative strain that has improved lipid productivity with respect to a microorganism of the progenitor strain. In certain examples the method can comprise: subjecting microorganisms of a progenitor strain to a mutagenesis procedure, culturing the mutagenized microorganisms in a cytostat or chemostat in the presence of an inhibitor of an enzyme or factor that participates in lipid biosynthesis for a period of time during which the microorganism undergoes multiple cell divisions, to provide an inhibitor-selected population of microorganisms, subjecting at least one mutagenized microorganism to UV irradiation, and isolating at least one microorganism of a derivative strain of the progenitor microorganism strain that has improved lipid productivity with respect to the progenitor strain. The mutagenized microorganism can be treated with UV irradiation before and/or after selection in a cytostat or chemostat that includes an inhibitor of an enzyme or factor that participates in lipid biosynthesis. Selection in a cytostat or chemostat, in the presence or absence of an inhibitor of an enzyme or factor that participates in lipid biosynthesis, can also be performed after UV treatment.

Described herein are methods for improving traits of mutagenized strains in which a microorganism of a progenitor strain is subjected to UV treatment after the mutagenesis procedure. Without limiting to any specific mechanism, UV treatment may enhance a trait of a mutagenized strain. UV treatment can occur after mutagenesis and before or after selection or screening for a trait of interest. In some examples, selection for a trait of interest can include selection in a cytostat or chemostat. Alternatively or in addition, selection or screening for a trait of interest can include growth assays (including tests for resistance to one or more compounds), biochemical analysis, genetic analysis, or phenotypic, cellular, or biochemical assays.

In some examples, selection or screening for a trait of interest occurs prior to UV treatment. Selection for a trait of interest can optionally include culturing the mutagenized microorganism in a cytostat or chemostat. In some examples, selection for a trait of interest can include culturing the mutagenized microorganism in a cytostat or chemostat that includes at least one compound that inhibits growth of wild type cells or, for example, inhibits the activity of an enzyme or factor that participates in lipid metabolism. A mutagenized microorganism can alternatively or in addition be screened or characterized, for example, by biochemical analysis or cellular or biochemical assays, to assess the trait of interest, for example, productivity related to any compound or group or class of compounds, resistance to compounds, temperature tolerance, growth rate, etc.

A microorganism or strain can be subjected to multiple mutagenesis procedures that optionally but preferably can each be followed by UV treatment, and optionally multiple mutagenesis procedures are followed by selection of microorganisms for a trait of interest before and/or after UV treatment.

The methods provided herein can be performed on any microbial strain of interest, and can be, as nonlimiting examples, a heterokont, an alga, a fungus, or a bacterium. The strain selected for a trait of interest, including but not limited to increased lipid production, can be a recombinant strain or a non-recombinant strain. For example, the strain may be of a species not known to produce significant or substantial amounts of a lipid of interest, but may be engineered to express one or more genes that increase lipid production or result in production of a particular lipid or class of lipids. Microorganisms treated with UV can be from single cell isolated, or can be populations of cells that have been subjected to a mutagenesis procedure. Microorganisms treated with UV following a mutagenesis procedure (which can be, for example, treatment with a mutagenic compound, gamma irradiation, or UV irradiation) can be microorganisms that have been subjected to one or more mutagenesis procedures and/or one or more screens or selection procedures prior to the selection procedure.

Microorganisms used in the methods herein can include any microorganisms, including prokaryotes and eukaryotes. Where the methods are used to obtain strains with enhanced lipid production, species that naturally accumulate lipids may be preferred, although the disclosure is not limited to such species. A microorganism used in any of the methods herein can be, in some examples, a heterokont strain of the Labyrinthulomycete class, and can be, for example, a Thrustochytrid or Labyrinthulid, such as a species of any of the genera *Aplanochytrium, Aurantiochytrium, Diplophrys, Japonochytrium, Labyrinthula,* Labyrinthuloides, *Oblongichytrium, Schizochytrium, Thraustochytrium,* or *Ulkenia.*

In further examples, a microorganism can be an alga, such as, for example, a microalga such as for example, a species of a genus selected from the group consisting of *Achnanthes, Amphiprora, Amphora, Ankistrodesmus, Asteromonas, Boekelovia, Bolidomonas, Borodinella, Botrydium, Botryococcus, Bracteococcus, Chaetoceros, Carteria, Chlamydomonas, Chlorococcum, Chlorogonium, Chlorella, Chroomonas, Chrysosphaera, Cricosphaera, Crypthecodinium, Cryptomonas, Cyclotella, Dunaliella, Ellipsoidon, Emiliania, Eremosphaera, Ernodesmius, Euglena, Eustigmatos, Franceia, Fragilaria, Fragilaropsis, Gloeothamnion, Haematococcus, Halocafeteria, Hantzschia, Heterosigma, Hymenomonas, Isochrysis, Lepocinclis, Micractinium, Monodus, Monoraphidium, Nannochloris, Nannochloropsis, Navicula, Neochloris, Nephrochloris, Nephroselmis, Nitzschia, Ochromonas, Oedogonium, Oocystis, Ostreococcus, Pavlova, Parachlorella, Parietochloris, Pascheria, Pelagomonas, Phaeodactylum, Phagus, Picochlorum, Platymonas, Pleurochrysis, Pleurococcus, Prototheca, Pseudochlorella, Pseudoneochloris, Pseudostaurastrum, Pyramimonas, Pyrobotrys, Scenedesmus, Schizochlamydella, Skeletonema, Spyrogyra, Stichococcus, Tetrachlorella, Tetraselmis, Thalassiosira, Tribonema, Vaucheria, Viridiella, Vischeria,* and *Volvox.* For example, a diatom such as, but not limited to, a species of *Cyclotella, Paeodactylum,* or *Thalassiosira* may be used. Alternatively, a dinoflagellate such as *Crypthecodinium* can be used. Alternatively, a eustigmatophyte such as a species of *Monodus* or *Nanochloropsis* can be used in the methods provided herein.

In yet other examples, a microorganism selected for a trait of interest, such as enhanced lipid production, can be a recombinant or nonrecombinant fungus, such as, for example, a species of *Aspergillus, Mortierella, Mucor, Saccharomyces, Schizosaccharomyces,* or *Pichia.* In some examples, a microorganism selected for increased lipid production is an oleaginous yeast, for example, a species of *Candida, Cryptococcus, Cunninghamella, Lipomyces, Rhodosporidium, Rhodotortula, Thamnidium, Trichosporon,* or *Yarrowia.* Nonlimiting examples of species of oleaginous fungi that may be considered for selection for increased lipid production include *Aspergillus nidulans, Cryptococcus curvatus, Cunninghamella echinulata, Debaryomyces hansenii, Geotrichum histeridarum, Geotrichum vulgare, Lipomyces orientalis, Lipomyces starkeyi, Lipomyces tetrasporus, Mortierella alpine, Mortierella ramanniana, Rhodosporidium sphaerocarpum, Rhodotorula aurantiaca, Rhodotorula glutinis, Rhodotorula mucilaginosa, Rhodotorula terpendoidalis, Rhodotorula toruloides, Sporobolomyces alborubescens, Thamnidium ctenidium, Thamnidium elegans, Torulaspora delbruechii, Trichosporon behrend, Trichosporon brassicae, Trichosporon sp. CBS 7617, Trichosporon domesticum, Trichosporon loubieri, Trichosporon montevideense,* and *Yarrowia lipolytica.*

A strain selected for a trait of interest such as increased lipid production can also be a recombinant or nonrecombinant bacterium. For example, bacteria known to produce triglycerides that can be selected for increased lipid production can include species of *Acinetobacter, Mycobacterium, Nocardia, Rhodococcus, Shewanella,* and *Streptomyces.* Cyanobacterial genera that may be considered include *Agmenellum, Anabaena, Anabaenopsis, Anacystis, Aphanizomenon, Arthrospira, Asterocapsa, Borzia, Calothrix, Chamaesiphon, Chroococcus, Chlorogloeopsis, Chroococcidiopsis, Chroococcus, Crinalium, Cyanobacterium, Cyanobium, Cyanocystis, Cyanospira, Cyanothece, Cylindrospermopsis, Cylindrospermum, Dactylococcopsis, Dermocarpella, Fischerella, Fremyella, Geitleria, Geitlerinema, Gloeobacter, Gloeocapsa, Gloeothece, Halospirulina, Iyengariella, Leptolyngbya, Limnothrix, Lyngbya, Microcoleus, Microcystis, Myxosarcina, Nodularia, Nostoc, Nostochopsis, Oscillatoria, Phormidium, Planktothrix, Pleurocapsa, Prochlorococcus, Prochloron, Prochlorothrix, Pseudanabaena, Rivularia, Schizothrix, Scytonema, Spirulina, Stanieria, Starria, Stigonema, Symploca, Synechococcus, Synechocystis, Tolypothrix, Trichodesmium, Tychonema* and *Xenococcus.*

### Cytostat and Chemostat Selection

The present disclosure includes methods for selecting variants that have increased tolerance for lipid biosynthesis inhibitors or other compounds affecting lipid biosynthesis. The selection procedures can employ a chemostat or cytostat to select for one or more derivatives or mutants with increased tolerance to compounds that inhibit lipid biosynthesis while enriching the culture for variants that grow most rapidly under the selective condition.

A chemostat is a bioreactor that includes a medium, which may be, for example, a selective medium (*e.g*., a medium that includes a compound or nutrient source that impairs or limits the growth of non-resistant cells or non-nutrient source utilizing cells), where one nutrient in the culture medium is present in a limiting amount. The culture grows until the level of the limiting nutrient drops to a level where cell division slows. A constant inward flow of fresh medium is maintained at a rate equal to a constant outward flow of the cell culture. In this way, the culture is continuously diluted and continuously grows (divides) where the growth (rate of cell division) of the culture is directly related to the rate of culture dilution. Thus, the rate of cell division can be adjusted by adjusting the culture dilution rate, and variants that have a growth advantage under the selective condition become enriched in the culture as poorer growing cells "wash out" through continuous dilution of the culture.

A cytostat is similar to a chemostat, except that the culture is not allowed to approach nutrient limitation (the culture is maintained at a density below that at which nutrient limitation would occur), and dilution of the cytostat occurs based on cell density of the culture, which is monitored at regular intervals or even continuously, for example, by a flow cytometer that can be integral to the cytostat apparatus (see, for example, US Patent No. 7,901,937). Because cytostat cultures do not approach nutrient limitation (or experience any significant accumulation of fermentation products that may affect growth), cytostat cultures are considered to be in a steady state of growth. As in chemostat cultures, in a cytostat that includes selective media, cells having the best growth rates under the selective conditions become enriched in the culture, as poorly growing variants of the culture become scarcer and scarcer through culture dilution. In the cytostat, mutants increase their representation in the culture quickly, as nutrients are not limiting so that mutants having a growth advantage divide more rapidly without being periodically impeded by nutrient depletion. Moreover, because the dilution rate is calibrated to the cell division rate, cells are allowed to proliferate without excessive dilution until cells begin to achieve a certain density, generally resulting from the emergence of resistant mutants in the culture. This continuous enrichment process where dilution is directly tied to cell density of the culture greatly reduces the time of selecting for variants that are favored by the cytostat conditions.

Although the production of lipid by microorganisms such as chytrids and oleaginous yeasts is highly induced during nutrient limitation (typically nitrogen limitation) when cell division does not occur, it was found that a chemostat/cytostat selection process that relies on higher rates of cell division to isolate mutants of interest could nonetheless be successfully employed to isolate mutants having enhanced lipid production.

As used herein "inhibits" means to reduce the activity of an enzyme or other factor by any means. By "factor that participates in lipid metabolism" is meant any molecule whose presence or amount enables, induces, suppresses, increases, or decreases lipid biosynthesis in the cell. A factor that participates can be a protein or peptide, including, for example a protein that directs or increases biosynthesis of enzymes that participate in lipid biosynthesis or degradation, or a protein that regulates the activity of other proteins enzymes that participate in lipid biosynthesis or degradation, for example, a transcription factor, a kinase or phosphatase, or a protein or peptide enzyme inhibitor or allosteric regulator. A factor that participates in lipid metabolism can also be, for example, a carrier protein or a transporter. A factor that participates in lipid metabolism need not be a protein or peptide, but can be, for example, a small molecule cofactor, pathway intermediate, transcriptional inducer or repressor compound, or lipid or nucleotide cofactor that directly or indirectly affects the activity of a protein, etc.

The chemostat or cytostat can include as inhibitor(s), for example, malic acid, one or more fatty acids, enzyme inhibitors, or other compounds that affect lipid production, such as, for example, alkyl galate, propyl galate, capsaicin, cerulenin, curcumin, cyclopropene, diphenylamine, norflurazon, pyridazinone, sethoxydim, toluic acid, triclosan, haloxyfop, diclofop, fenoxaprop, quizalofop, 6-aminonicotinamide, malate, one or more fatty acids (e.g., oleic acid), canola oil, peanut oil, or sesamine or sesamol. Any or a combination of compounds that may affect lipid biosynthesis may be employed. In some instances, an inhibitor of fatty acid synthase (FAS), or a component thereof, may be provided in a chemostat or cytostat culture. For example, an inhibitor may be an inhibitor of a dehydratase, enoyl-ACP reductase, or beta-keto acyl synthase activity of a FAS. Hydroxy acyl carrier protein dehydratase ("dehydratase" or "DH") inhibitors include 3-decynoyl-N-acetyl-cysteamine (3-decynoyl-NAC) and derivatives thereof (Ishikawa et al. (2012) J. Amer. Chem. Soc. 134:769-772). Inhibitors of enoyl-ACP reductase ("ER") activity include, as non-limiting examples, diazaborines, triclosan, and isoniazid. Inhibitors of beta-ketoacyl-ACP synthase ("KAS") activity include, as non-limiting examples, cerulenin, thiolactomycin, and SBPT04 (Kingry et al. (2012) J. Bacteriol. 195:351-358). Inhibitors of the enzyme acetyl-CoA carboxylase (ACCase) enzyme include, without limitation, haloxyfop, diclofop, fenoxaprop, quizalofop, BP1, TOFA, and soraphen A (Becker *et al.* (2007)).

Also considered are inhibitors of fatty acid desaturases and fatty acid elongases, such as, for example, fatty acid desaturase inhibitors such as sesamine or curcumin (US 8,349,595), or fatty acid elongase inhibitors such as cycloate, thiocarbamates, and sulfoxide.

In some examples, triclosan may be used as an inhibitor in the chemostat or cytostat. In some examples, cerulenin may be used as an inhibitor in the the chemostat or cytostat. Additionally, any strains of interest, such as strains isolated from the wild or strains obtained from culture collections can optionally be cultured in a chemostat or cytostat in the absence of an inhibitor of an enzyme or other factor that participates in lipid metabolism to select isolates having more rapid growth than the original strain. Such selections can be done prior to or following a selection for mutants having resistance to an inhibitor compound.

Selection in a cytostat or chemostat, with or without a lipid biosynthesis inhibitor, can be for any period of time that allows for multiple successive cycles of cell division, and may depend on the growth rate of the organism. The chemostat or cytostat can be of any volumetric capacity. For microbial strains such as fungi and chytrids, for example, culturing can be in a chemostat or cytostat with a fermenter volumetric capacity of from about 25 mL to about 10 L, and can be from 200 mL to about 5L, or from about 300 mL to about 2L, or from about 400 mL to about 1L. The culture period can be for any length of time, such as for example, from one day to several months. Preferably, for fungi and chytrids, the culturing period in a chemostat or cytostat is for a period of time greater than one day, for example, for a period of from about 2 days to about 30 days, or from about 3 days to about 20 days, or from about 4 days to about 15 days. In some instances cytostat selection is preferred. In a cytostat, the culture period can in some instances, for example, be from about 3 days to about 10 days.

Single colonies of the microorganism can be isolated by dilution plating or flow cytometry from cultures grown for any period of time and the resulting isolate or isolates can be screened for any desirable properties.

For example, an isolate can be tested for any one of or any combination of: increased growth rate, increased biomass accumulation, increased lipid or fatty acid production rate, increased triglyceride production rate, increased total lipid accumulation, increased FAME accumulation, increased triglyceride accumulation, increased FAME production rate, increased DHA production rate, increased DHA accumulation, increased DHA as a percentage of fatty acids, increased ratio of DHA to DPA, etc. Any feasible methods for determining lipid or fatty acid amounts can be employed, including the use of lipophilic dyes (*e.g*., Bodipy or Nile red) or FAME analysis. The testing can be under any culture conditions, including those listed hereinabove that may be used in culturing a strain prior to testing, for example, using particular carbon or nitrogen sources or concentrations, salt concentration, temperature, pH, etc.

As used herein, "mutants" includes microorganisms that have incurred a mutation, *i.e.,* a change, in a gene. A mutant can be naturally occurring (*i.e.,* can arise spontaneously) or can be induced, for example, using chemical agents (including drugs), gamma irradiation, or ultraviolet light. The term variant is typically used to encompass mutants that arise spontaneously, for example, an isolate that has traits that are distinct with respect to the strain from which it arose (the progenitor strain) that are stable and heritable, although the nature of the mutation or even the gene(s) or protein(s) responsible for the distinctive trait(s) may be unknown. A "derivative" strain is a strain that is a descendent of a progenitor strains that has been subcultured and maintained separately from a progenitor strain. A derivative may be a variant or mutant strain, for example, a variant or mutant strain having one or more distinctive heritable traits with respect to the progenitor strain. A derivative strain may also be a strain that has been genetically engineered to include at least one non-native gene, such as, for example, a non-native gene encoding a metabolic enzyme, a regulator (*e.g.,* a transcription factor, transcriptional activator, allosteric protein, transporter, etc.)

### Mutagenesis

The disclosure includes variants of any of the strains provided herein, where a variant can be a mutant, such as a naturally-occurring mutant, or a mutant generated by any of a large number of mutagenesis techniques, such as but not limited to, chemical mutagenesis, gamma irradiation, UV irradiation, or molecular biology techniques using a nucleic acid construct introduced into the cell that can be used (directly or indirectly) to alter a genetic locus of a microbial cell, such as, but not limited to, insertional mutagenesis, site-directed mutagenesis, gene replacement or gene excision. Also disclosed are methods for selecting variants (including mutants) of a microbial strain in which the methods may include one or more mutagenesis steps, such as but not limited to, those provided herein.

Methods for generating mutants of microbial strains are well-known. For example, gamma irradiation, UV irradiation, and treatment with any of a large number of possible chemical mutagens (e.g., 5-bromo deoxyuridine, ethyl methane sulfonate (EMS), methyl methane sulfonate (MMS), diethylsulfate (DES), nitrosoguanidine (NTG), ICR compounds, etc.) or treatment with compounds such as enediyne antibiotics that cause chromosome breakage (e.g., bleomycin, adriamycin, neocarzinostatin) are methods that have been employed for mutagenesis of algae, fungi, and chytrids (see, for example, US Patent 8,232,090; US Patent Application 20120088831; US Patent Application 20100285557; US Patent Application 20120258498. Among the physical mutagens, electromagnetic radiation comprising radioactive radiation, gamma-rays and x-rays, ionizing radiation, ultraviolet-light or elevated temperature. A large number of chemical mutagens are also known in the art including but not limited to, intercalating agents, alkylating agents, deaminating agents, base analogs. Intercalating agents include, as nonlimiting examples, the acridine derivatives or the phenanthridine derivatives such as ethidium bromide (also known as 2,7-diamino-10-ethyl-6-phenylphenanthridium bromide or 3,8-diamino-5-ethyl-6-phenylphenantridinium bromide). Nonlimiting examples of alkylating agents include nitrosoguanidine derivatives (e.g., N-methyl-N'-nitro-nitrosoguanidine), ethyl methanesulfonate (EMS), ethyl ethanesulfonate, diethylsulfate (DES), methyl methane sulfonate (MMS), nitrous acid, or HNO₂, and the nitrogen mustards or ICR compounds. Nonlimiting examples of base analogs that can be used as mutagens include the compound 5-bromo-uracil (also known as deoxynucleosid 5-bromodeoxyuridine), 5-bromo deoxyuridine, and 2-aminopurine.

Mutagenesis can also employ molecular biological techniques, including introduction of exogenous nucleic acid molecules into the microbial cell of interest. For example an exogenous nucleic acid molecule introduced into the cell can integrate into a genetic locus by random or targeted integration, affecting expression of genes into which the foreign DNA inserts or genes that are proximal to foreign DNA inserted into the genome *(e.g.,* US Patent 7,019,122; US Patent 8,216,844). The exogenous nucleic acid molecule can, for example, include a transposable element or a component thereof, such as, for example, inverted repeats that can be recognized by a transposase and/or a gene encoding a transposase, or the exogenous nucleic acid molecule can be based at least in part on a virus, such as an integrating virus, for example, a retrovirus.

The microorganisms to be mutagenized can be exposed to a mutagen or a mutation inducing agent according to any known methods. In general mutagenesis can be carried out by exposing a suitable quantity of cells of the microbial strain of interest to a mutagen or mutation-inducing agent within an appropriate time and conditions. Often, one or more preliminary experiments are performed in which the microbial strain of interest is exposed to the mutagen or mutation-inducing gene to determine a concentration range that is effective in generating mutants and does not kill an excessive number of cells. Such procedures are well known in the art. For example, on the basis of the results obtained through such experiments it could be shown that a concentration and/or duration of exposure that allows for viability of cells between about 0.5% and about 95%, or between about 1% and about 90%, or between about 2% and about 80%, or between about 5% and about 70%, or between about 10% and about 50% can be used in mutagenesis procedures. The time of exposition of the cells to the mutagen depends on the nature of the mutagen to be used. The time of contacting the cells with the mutagen can be, for example, between about 1 minute and about 24 hours, or between about 30 minutes and about 6 hours, or between about 1.0 and about 3.0 hours. It can be advisable to conduct a series of simple tests in order to determine the viability rate of the cells to be mutagenized after various treatments with the mutagen.

For purposes of example, and not by way of limitation, mutagenesis can be performed by suspending cells to be mutagenized in a sterilized liquid medium or in sterilized water that can include, for example, a chemical mutagen or mutagenic compound, in an amount between 10⁵ to 10¹⁰ cells per ml, for example exhibiting a density of about 10⁶ - 10⁸ cells per ml, or by spreading them on an agar plate and exposing them to the mutagen (an agar plate can be used, for example, for gamma or UV irradiation). Mutagenesis of the microbial cells can be performed with the culture in culture flasks or tubes, and can be performed at any temperature at which the microbe of interest is viable.

After treatment with a chemical mutagen, the cells can be washed at least once with sterile water or medium or diluted into a medium that does not include the mutagen. The cells can be cultured in the absence of the mutagen and in the absence of any selective agent in a recovery period prior to culturing in a cytostat or chemostat in the presence of a selective agent.

After the cytostat or chemostat culturing period, which may be on the order of days to weeks, cells may be plated at dilution or cell sorted for single colony isolation. Colonies can be grown in liquid culture, for example, to test for production of lipid or growth rate.

Two or more mutagenesis and cytostat or chemostat selection procedures can be performed in tandem. The successive cytostat or chemostat incubations can include different inhibitors (e.g., lipid biosynthesis inhibitors that inhibit different enzymes on the pathway). The mutagenesis procedures can use the same or different mutagens. In a particular example, cells that have experiences one or more mutagenesis procedures are further treated with UV and selected in a cytostat that does not include a lipid biosynthesis inhibitor.

### UV Treatment

Provided herein are methods for improving the performance of a microorganism by treating the microorganism with UV light, culturing the UV-treated microorganism, and screening for a desired trait. By "improving the performance of" is meant, for example, improving growth rate, increasing productivity, enhancing resistance to or tolerance of chemicals, pH, salt, *etc.* UV irradiation can be performed after a mutagenesis procedure, for example, a mutagenesis procedure that uses chemicals or gamma irradiation, or a microorganism can be treated with UV after a separate UV mutagenesis step. Optionally, a selection can be performed after a mutagenesis step and before exposure of the cells to UV radiation.

For example, a mutagenesis procedure can be performed, and the population of cells that have been treated with a mutagen can be selected for the presence or degree of a certain trait, such as, for example, higher growth rate, higher rate of production of a product, absolute or relative amount of one or more specific products produced, resistance or tolerance of the cells to a compound or growth condition, etc. Cells that are selected in the screen as having the desired trait are subsequently treated with UV irradiation. Preferably, the cells are then tested again for the presence or degree of the trait to identify clones having an enhanced or improved trait, such as, for example, higher growth rate, higher rate of production of a product, greater resistance to or tolerance of a compound or growth condition, etc.

Following UV irradiation, and prior to selection, cells are optionally protected from light. For example, the cells may be plated following UV treatment and the plates may be kept in a dark place, such as a cabinet, or may be wrapped in light-impermeable material. The cells may be kept in the dark for from one half hour to thirty days, for example, from one hour to two weeks, or from two hours to one week.

Without limiting to any particular mechanism, it is speculated an isolated mutant or variant that exhibits a certain trait that is distinct from wild type can exhibit enhanced traits following UV treatment.

UV treatment can use well-known methods of UV mutagenesis, and can use, for example, UV lamps or cross-linker devices used for nucleic acid and protein UV crosslinking, and the intensity and duration of treatment can be tailored such that, for example, between about 0. 1% and about 99.99% of the cells are killed by the treatment, for example, at least 0.5%, at least 1%, at least 2%, at least 5%, at least 10%, at least 20%, at least 30%, at least 40%, at least 50%, at least 55%, at least 60%, at least 65%, at least 70%, at least 75%, at least 80%, at least 85%, at least 90%, at least 95%, or at least 99% of the population of microorganisms receiving the UV treatment may be killed by the treatment. UV irradiation between about 100 µJ/cm²and about 50,000 µJ/cm² can be used, such as between about 300 µJ/cm²and about 30,000 µJ/cm², or between about 500 µJ/cm² and about 15,000, for example, at an energy level of at least about 1000, 3000, 5000, 7000, or 10000 µJ/cm², can be performed.

The UV treated microorganism can optionally be incubated in the dark after UV treatment for a period of from about 15 minutes to about two weeks or more, for example, from about 30 minutes to about one week. The population of cells can be selected after being maintained in the dark, and, optionally but preferably, can be selected after being maintained in the dark and then cultured for an additional period of time. For example, cells can be UV treated, incubated in the dark, and then selected or screened, for example, the UV treated cells may be selected by growth in a cytostat or chemostat that can optionally include a selective compound, such as, for example, a lipid biosynthesis inhibitor. Alternatively, after UV treatment, cells can be maintained in the light without providing a dark incubation period. The cells can be selected immediately after UV treatment, or a "grow out" culturing period can be performed prior to selection. The culturing period occurring after UV treatment can optionally be in a cytostat or chemostat, with or without a selective compound, such that cells with the best growth characteristics are selected during the grow out period.

In some instances, cells may be UV treated after a first, second, third, or subsequent mutagenesis procedure and, optionally but preferably, screen or selection, such as a cytostat or chemostat selection. For example, cells can be treated with a mutagen, such as a chemical mutagen or gamma or UV irradiation, and then selected in a chemostat or cytostat (preferably, where the trait of interest is lipid production, including an inhibitor of an enzyme or factor that participates in lipid biosynthesis). Isolates from the one or more mutagenesis and selection procedures can be screened or selected for having a desired trait, such as, for example, enhanced growth rate or lipid production rate, or enhanced resistance to or tolerance of particular chemicals, etc. can be treated with UV, for example, as described herein, and then (optionally but preferably following a dark incubation period) optionally selected in a cytostat or chemostat that may or may not include an inhibitor or selective agent to isolate a mutant having enhanced properties (such as, for example, lipid biosynthesis) with respect to the previously mutagenized strain. Cells may also in an alternative be UV treated after a first, second, third, or subsequent mutagenesis procedure and selection, and then plated on plates that include a compound that inhibits lipid biosynthesis to isolate mutants having enhanced traits, such as enhanced lipid production.

UV irradiation can be performed after any mutagenesis procedure, for example, a mutagenesis procedure that uses chemicals or gamma irradiation, or a microorganism can be treated with UV after a separate UV mutagenesis step. Optionally, a selection can be performed after a mutagenesis step and before exposure of the cells to UV radiation. For example, UV treatment can be performed after only one, some, or all mutagenesis procedures in a strain isolation process that uses multiple mutagenesis steps.

### EXAMPLES

### Example 1. Isolation of wild-type labyrinthulomycete strains.

A collection project that isolated hundreds of microorganisms for assessing lipid production was initiated. Wild type strain isolation biotopes for sampling were identified based upon access via legal permits and the known biology of the class of organism. Biotopes were categorized as open ocean, estuary, coastal lagoon, mangrove lagoon, tide pool, hypersaline, freshwater, or aquaculture farm. Sampling location latitudes spanned the range from temperate, subtropical to tropical. Water samples collected included direct samples of 2 liters. In some cases, plankton tows were performed using a 10 µm net. A total of 466 environmental samples were collected from 2010-2012. Temperature ranged from 4°C to 61°C, and pH ranged from 2.45 to 9.18. Dissolved oxygen ranged from 0 to 204% air saturation and salinity ranged from 0 ppt to 105 ppt. All samples were inoculated on site into 125 f/2 media (composition: 75 mg/L NaNO₃, 5 mg/L NaH₂PO₄·H₂O, 0.005 mg/L biotin, 0.01 mg/L CoCl₂.6H₂O, 0.01 mg/L CuSO₄.5H₂O, 4 mg/L Na₂EDTA, 3 mg/L FeCl₂, 0.18 g/L MnCl₂, 0.006 mg/L Na₂MoO₄.2H₂O, 0.1 g/L thiamine, 0.005 mg/L vitamin B12, 0.022 mg/L ZnSO₄) and seawater-glucose-yeast-peptone (SWGYP) media (composition: 2 g glucose, 1 g peptone and 1 g Difco yeast extract per liter of sterile-filtered seawater) to initiate growth. A separate set of samples was generated in duplicate 50 ml aliquots that included 10% glycerol and subsequently frozen with dry ice. Finally, additional samples were frozen in 10% glycerol in a volume of 2 L for subsequent DNA isolations. The samples were shipped to the laboratory on the day of collection and arrived the following day for inoculation into fermentation broth in stationary or shake flasks. Carbenicillin, streptomycin, and nystatin were included in the cultures to retard the growth of bacteria or fungi.

Inoculated enrichments were incubated at a range of temperatures from 15°C to 30°C and subsequently plated onto SWGYP or f/2 agar with antibiotics. Isolated colonies were recovered, amplified in SWGYP or f/2 media and PCR amplification of 18s rDNA was performed to determine taxonomic identity of the isolated microorganism.

### Example 2. Comparison of lipid productivity of isolated strains

Isolated labyrinthulomycete strains were screened for lipid productivity using a Micro24 miniature bioreactor system (Pall Life Sciences, Port Washington, NY). Briefly, 6 ml cultures were established containing a culture medium containing 5g/l yeast extract, 1.65 g/l (NH₄)₂SO₄, 0.5 g/l KCl, 2.5 g/L MgSO₄, 8g/l Instant Ocean salts (Aquatic Eco Systems, Apopka, FL), 5 ml Trace Elements solution, 1 ml/L Vitamin solution, and 20 g/l dextrose. The trace element solution contained 6 g/l EDTA di-sodium salt; 0.29 g/l FeCl₃.6H₂O; 6.84 g/l H₂BO₃; 0.86 g/l MnCl₂.4H₂O; 1 ml ZnCl₂ stock solution (60 g/1); 1 ml CoCl₂.6H₂O stock solution (2g/l); 1 ml NiSO₄.6H₂O stock solution (60 g/l); 1 ml CuSO₄.5H₂O stock solution (2g/l); and 1 ml Na₂MoO₄.2H₂O stock solution (5g/l). The vitamin solution contained 200 mg/l thiamine, 10 ml per liter of a 0.1 g/l biotin stock solution; and 1 ml per liter of a 1g/l stock solution of cyanocobalamin. Ten microliters of 3% silicone antifoam were added to each 6 ml culture. The cultures were inoculated with 600 microliters of a mid-log phase culture of each strain and incubated for fourteen hours at 30°C at a pH of about 6.8, under 600 rpm agitation. The dissolved oxygen concentration was 10% of saturation.

At the end of the test period, cells were harvested and aliquots were analyzed for biomass and FAME. For biomass assessment, 4 ml of fermentation broth was pipetted to a pre-weighed 15 ml falcon tube. The tube containing the culture aliquot was centrifuged at 3220 x g for 20 min, and the supernatant was decanted. The pellet was then frozen at -80°C overnight, followed by freeze drying for 16-24 h. The falcon tube with dried pellet inside was weighed, and the weight of the lyophilized pellet was calculated by subtracting the weight of the empty tube. The lyophilized pellet weight was standardized by dividing by the aliquot volume (4 ml) to obtain a value for the biomass per ml of culture. Fatty acid methyl ester (FAME) was assessed by standard methods using gas chromatography to analyze the fatty acid content of triplicate 50 - 200 µL volume aliquots of the cultures. The culture aliquots were diluted 1:10 in 1X PBS prior to aliquoting and drying for FAME sample preparation. The samples were dried via HT-4X GeneVac and stored at -20°C until prepped for fatty acid methyl ester analysis. For extraction, 0.5 mL of 5M potassium hydroxide in methanol and 0.2 mL tetrahydrofuran containing 25 ppm butylated hydroxy toluene were added to the samples. Next 40 uL of a methyl ester internal standard mix that included 2 mg/mL C11:0 free fatty acid, 2 mg/mL C13:0 triglyceride, and 2 mg/mL C23:0 fatty acid in n-heptane was added. After about 0.5mL of 425-600 µm acid washed glass beads were added, and the samples were placed into a GenoGrinder at 1200 rpm for 7 min. The samples were then heated at 80° C for 5min and this was followed by 5 min at 2500 rpm on a multi-tube vortexer. Methanol containing14% boron trifluoride was then added to the samples and they were returned to the 80° C heating block for 30 min. The samples were then vortexed once again at 2500 rpm for 5 min. Lastly, 2 mL of n-heptane and 0.5mL 5M sodium chloride were added and the samples were vortexed a final time at 2500 rpm for 5min. The samples were shaken vigorously to ensure emulsion. The racks were then centrifuged at 1000 rpm for 1 min after which the top layer was sampled by a Gerstel MPS autosampler paired to a 7890 Agilent GC equipped with a flame ionization detector.

Wild-type strains "886" and "1602" were selected in this screen as high DHA-producing strains, as determined by the % DHA of total FAME lipids produced and the DHA productivity rate (calculated as mg DHA produced per liter per hour) (**Table 2**). The strains were renamed WH-5628 ("886") and WH-5554 ("1602").

**Table 2. Productivity of Labyrinthulomycete Isolates in Small Scale Fermentation**

| **Strain** | **886** | **25** | **94** | **1473** | **1578** | **1439** | **1478** | **1514** | **1602** |
|---|---|---|---|---|---|---|---|---|---|
| **Biomass (g/l/h)** | **0.51±0.2** | 0.37±0.1 | 0.33±0.1 | 0.39±0.2 | 0.39±0.1 | 0.42±0.2 | 0.51±0.1 | 0.59±0.1 | **0.62±0.02** |
| **DHA (mg/l/h)** | **49.0±14** | 30.4±3 | 42.2±8 | 40.1±15 | 32.5±18 | 28.3±13 | 41.4±2.8 | 69.9±4.6 | **71.3±1.8** |

### Example 3. Cytostat selection of chytrid variants with enhanced growth rate.

Strain 1602 (WH-05554) was used to inoculate a 0.5L cytostat with minimal medium containing 17 g/l Instant Ocean salts (Aquatic Eco Systems, Apopka, FL), 10 g/l dextrose, 1.65 g/l ammonium sulfate, 1 g/l monobasic potassium phosphate and 0.5 g/l potassium chloride. The cytostat consisted of an Infors fermenter (Bottmingen/Basel, Switzerland), an MSP flowcytoprep (Shoreview, MN) and an Accuri cytometer. The fermentation was kept at 30° C, and the pH was kept stable at pH 5.8 by addition of 0.5 M sodium hydroxide and 0.5 M phosphoric acid. The cell concentration was kept constant at 500,000 cells per ml over the course of 5 days. The dilution rate was initially 0.1/hr but increased to over 0.5/hr during day 4 and 5. On day 5 the fermentation was interrupted and an aliquot of the fermenter culture was plated. After incubating the plates at 30°C, colonies were selected and the resulting strain, 1602-RR01, was isolated from a single colony. The growth rate of strain 1602-RR01 in 1L fermenters (New Brunswick) was approximately 20% higher than the parent 1602 strain (**Table 3**), and it was therefore used as a baseline strain for subsequent mutagenesis experiments.

**Table 3. Growth rate of original 1602 isolate and cytostat selected derivative strain.**

| Strain | Growth Rate (h⁻¹) [from 2-4 h.] | Growth Rate (h⁻¹) [from 4-10 h.] |
|---|---|---|
| 1602, wt | 0.4212 | 0.2071 |
| 1602-RR01, cytostat | 0.4786 | 0.2871 |

### Example 4. Selection of a fast-growing cerulenin-resistant chytrid strain.

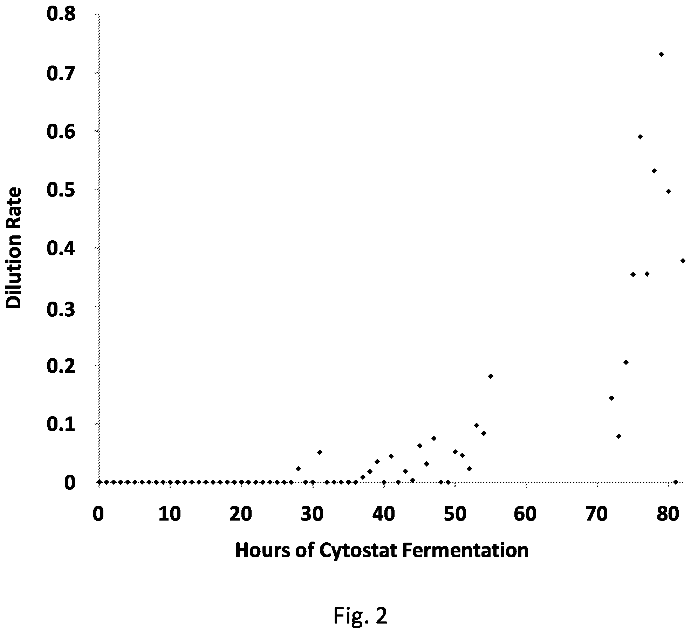

In separate treatments, four dosages of gamma radiation (25, 75, 100 and 150 Gy) were used to mutagenize 400 x 10⁶ cells of strain 1602-RR01, the isolate of strain WH-5554 selected for rapid growth (Example 3). The mutagenized cells were pelleted (2,000 x g for 5 min), resuspended in 5 ml of minimal medium and inoculated into the cytostat fermenter containing 500 ml of minimal medium with 3 µg/ml cerulenin (the cytostat setup was the same as in Example 3). Cerulenin is an inhibitor of the fatty acid synthase (FAS) that binds to the ketoacyl synthase domain (KAS) of the FAS enzyme (Price et al. (2001) J. Biol. Chem. 276:6551-6559). The dilution rate for the culture remained constant between 0 and 0.05 over the first 40 hours and then started to increase. At around 45 hours, the dilution rate increased to > 0.05 (**Figure 2**), indicating that cerulenin resistant variants started to become enriched. The media feed to the culture was interrupted between 56 and 68 hours allowing for higher cell density of the culture. At 68 hours the culture was manually diluted back to 0.5 million cells per ml with fresh medium that included 3 µg/ml cerulenin and cytostat function was resumed at 71 hours. The fermentation was interrupted at 82 hours at which point 5 ml of cells were subcultured into a 50 ml culture in a shake flask containing minimal medium without cerulenin. The culture was subcultured three times over the period of three days without cerulenin and was thereafter plated on agar plates containing 0, 2, 5, 10 and 20 µg/ml cerulenin. Growth was observed on all concentrations of cerulenin. Five colonies were picked from each plate for productivity evaluation in a Micro24 fermenter (Isett et al. (2007) Biotechnology and Bioengineering 98:1017-1028); Pall Life Sciences, Port Washington, NY). This bioreactor system allowed simultaneous testing of multiple small scale (6 ml) cultures that were incubated in replete minimal medium at 30°C for 23 hours. The minimal medium consisted of 1.65 g/l (NH₄)₂SO₄, 0.5 g/l KCl, 2.5 g/L MgSO₄, 17g/l Instant Ocean salts, 5 ml Trace Elements solution, 1 ml/L Vitamin solution, and 40 g/l dextrose. The trace element solution contained 6 g/l EDTA di-sodium salt; 0.29 g/l FeCl₃.6H₂O; 6.84 g/l H₂BO₃; 0.86 g/l MnCl₂.4H₂O; 1 ml ZnCl₂ stock solution (60 g/1); 1 ml COCl₂.6H₂O stock solution (2g/l); 1 ml NiSO₄.6H₂O stock solution (60 g/l); 1 ml CuSO₄.5H₂O stock solution (2g/l); and 1 ml Na₂MoO₄.2H₂O stock solution (5g/l). The vitamin solution contained 200 mg/l thiamine, 10 ml per liter of a 0.1 g/l biotin stock solution; and 1 ml per liter of a 1g/l stock solution of cyanocobalamin. The nitrogen in the incubation medium was present at an amount that was calculated to become depleted at around 8 hours of cultivation (following inoculation of the cultures with a starting OD 740 of 1.4), whereas glucose was provided in the medium at a level that allowed it to remain at replete levels all the way through the fermentation at 24 hours. Thus, the Micro24 culture medium was designed such that the strains being tested would undergo active growth for approximately 8 hours followed by a slowing and then cessation of growth and active lipogenesis for the following approximately 16 hours.

At the end of the 24 hour Micro24 culture period, aliquots of the cultures were removed for assessing biomass and fatty acids (FAME lipids) as provided in Example 3. Several derivative strains were observed to have increased FAME/TOC as well as a higher percentage of total organic carbon as DHA with respect to original strain 1602.

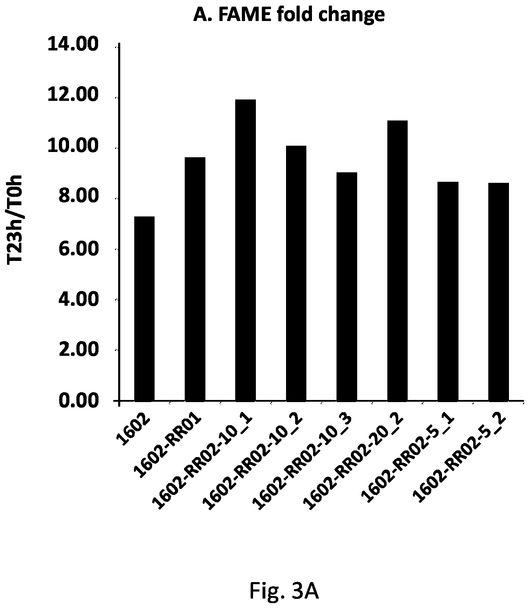

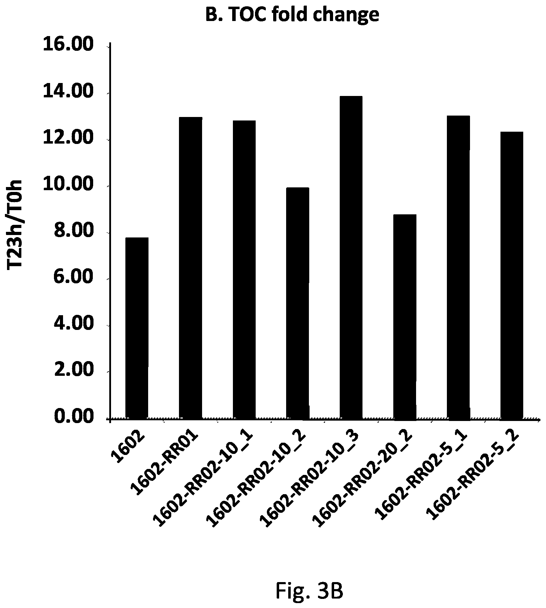

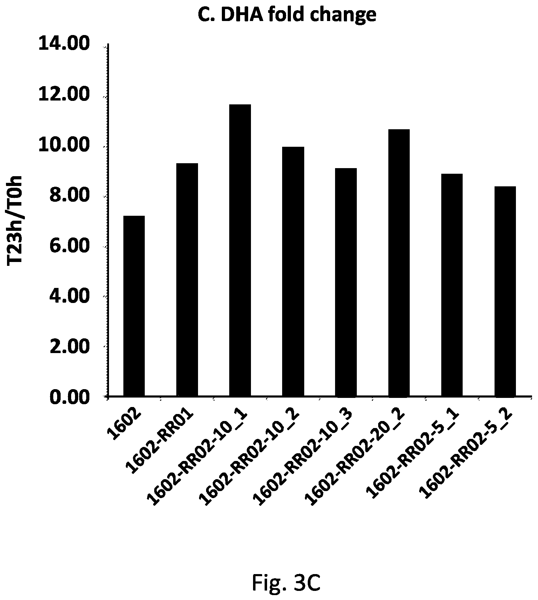

The fold change in TOC, FAME lipids, and DHA during the culturing period is provided in **Figure 3****.** Several derived strains were demonstrated to have had more rapid increases in total organic carbon, FAME lipids, and DHA over the 24 hour culture period than did the 1602 progenitor strain. Clone 1602-RR02-20-2 (isolated from the 20 µg/ml cerulenin agar plate) performed best in the Micro24 fermenter, demonstrating especially good FAME and DHA productivity. This cerulenin-resistant strain also outperformed the wild type strain in larger volume fermentations, both in terms of biomass and DHA productivity, and was given the designation NH-5574.

### Example 5. Selection of a fast-growing triclosan-resistant chytrid strain.

In separate treatments, five dosages of gamma radiation (25, 75, 100, 150 and 250 Gy) were used to mutagenize 400 x 10⁶ cells of strain NH-5574, the strain selected for cerulenin resistance in Example 4. The mutagenized cells were pelleted (2,000 x g for 5 min), resuspended in 5 ml of minimal medium, and inoculated into the cytostat fermenter containing minimal medium with 0.5 mg/l triclosan (the cytostat was set up as described in Example 3). Triclosan is an inhibitor of fatty acid synthase (FAS) which binds to the enoyl reductase domain (ER) of the enzyme (Heath et al (1999) J. Biol. Chem,. 274:11110-11114). The dilution rate of the cytostat culture remained constant between 0 and 0.05 over the first 55 hours but started to increase around 56 hours, when the dilution rate increased to > 0.5. The fermentation was interrupted at 115 hours at which point 5 ml of cells were subcultured into a 50 ml shake flask containing minimal medium without triclosan. The culture was subcultured three times over the period of three days without triclosan and was thereafter plated on agar plates containing 0, 0.5, 1 and 2 mg/l triclosan. Growth was observed on all concentrations of triclosan and a total of 20 colonies were picked from the plates for productivity evaluation in a Micro24 fermenter. Clone 1602-RR03-08 performed best in the Micro24 fermenter assay (as described in Example 4). **Table 4** demonstrates that the cerulenin + triclosan-resistant strain clearly outperformed the wild type strain both in DHA productivity. In addition, clone 1602-RR03-08, renamed strain NH-5783, also had a higher content of DHA as a percentage of total fatty acids (**Table 4**).

**Table 4. Productivity of labyrinthulomycete isolates in small scale fermentation**

| | **DHA (% FAME)** | **DHA productivity (g/L/h)** |
|---|---|---|
| **WH-5554** | 24.6±0.5 | 133±2.6 |
| **NH-5873** | 32.6±0.5 | 159.3.2±2.9 |

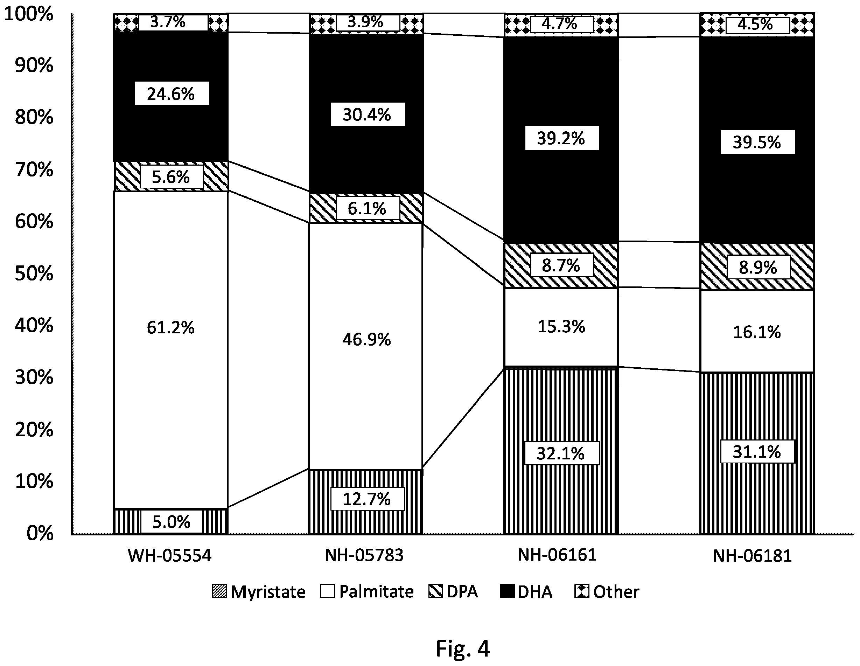

Depiction of an independent analysis of fatty acids produced by strains WH-5554 and WH-5783 is provided in **Figure 4****,** which shows WH-5783 has a higher percentage of FAME as DHA as compared to progenitor strain (31% as compared to 26%), while the ratio of DHA to DPA is approximately the same in strain NH-5783 (4.01) as in wild type progenitor strain WH-5554 (4.37). Also notable is a marked increase in myristic acid, which is more than twice the percentage of FAME in NH-5783 as in wild type progenitor strain WH-5554.

### Example 6. UV treatment of a strain with further enhancement of DHA production

Four hundred million cells of the NH-5783 strain isolated in Example 5 were plated on four 20 x 20 cm agar plates and the cells were allowed to attach for 2 hours. The four plates were exposed to four dosages of UV radiation (3000, 5000, 7000 and 10000 µJ/cm²) and allowed to recover for 2 hours in the dark, after which the cells were scraped off into 6 ml of minimal medium and injected into the cytostat fermenter containing minimal medium with no inhibitor (cytostat setup same as Example 3). The dilution rate was slow for the first 12 hours (∼0.2 but increased to close to wild type growth rates after that (> 0.5). Cells were removed from the cytostat after 24 hours and 120 hours of fermentation and single clones were isolated on agar plates. Nine clones from the 24 hour time point and 10 clones from the 120 hour time point were picked for productivity evaluation in a Micro24 fermenter. Clone 1602-RR13-04 (NH-6161) and 1602-RR13-10 (NH-6181), from the 24 hour and 120 hour time points respectively, performed best in the Micro24 fermenter. The results, provided in **Table 5** and depicted graphically in **Figure 4**, demonstrate that the UV-treated and cytostat selected produced an increased percentage of fatty acids as DHA (39% for NH-6161 and NH-6181 versus 30% for cerulenin/triclosan selected progenitor NH-5783 and just 24.6% for wild type progenitor WH-5554). In addition, strains NH- 6161 and NH-6181 had a considerably higher content of DHA and myristate (C14:0) that did progenitor strain NH-5783 as a percentage of total fatty acids (32% and 31% as compared with 12.7% for cerulenin-selected NH-5783 and just 5% for wild type WH-5554).

**Table 5. Fatty acid composition of Strains**

| | WH-5554 | NH-5783 | NH-6161 | NH-6181 |
|---|---|---|---|---|
| Myristate | 5.0% | 12.7% | 32.1% | 31.1% |
| Palmitate | 61.2% | 46.9% | 15.3% | 16.1% |
| DPA | 5.6% | 6.1% | 8.7% | 8.9% |
| DHA | 24.6% | 30.4% | 39.2% | 39.5% |
| Other | 3.7% | 3.9% | 4.7% | 4.5% |

### Example 7. Comparison of lipid productivity of wild-type isolated strain WH-5554 and classically improved derivative strains

Classically improved strains were screened for DHA productivity using a Micro24 miniature bioreactor system (Pall Life Sciences, Port Washington, NY). Briefly, 6 ml cultures containing a culture medium containing 1.65 g/l (NH₄)₂SO₄, 0.5 g/l KCl, 2.5 g/L MgSO₄, 17g/l Instant Ocean salts, 5 ml Trace Elements solution, 1 ml/L Vitamin solution, and 40 g/l dextrose were used. The trace element solution contained 6 g/l EDTA di-sodium salt; 0.29 g/l FeCl₃.6H₂O; 6.84 g/l H₂BO₃; 0.86 g/l MnCl₂.4H₂O; 1 ml ZnCl₂ stock solution (60 g/1); 1 ml CoCl₂.6H₂O stock solution (2g/l); 1 ml NiSO₄.6H₂O stock solution (60 g/l); 1 ml CuSO₄.5H₂O stock solution (2g/l); and 1 ml Na₂MoO₄.2H₂O stock solution (5g/l). The vitamin solution contained 200 mg/l thiamine, 10 ml per liter of a 0.1 g/l biotin stock solution; and 1 ml per liter of a 1g/l stock solution of cyanocobalamin.

The 6 ml cultures were normalized to a starting OD740 of 1.4 using mid-log phase culture of each strain from shake flasks. The Micro24 fermentations were incubated for 23 hours at 30°C, under 600 rpm agitation. Each strain was fermented in duplicate cultures. The dissolved oxygen concentration was 10% of saturation. At the beginning and end of the fermentation, cells were harvested and aliquots were analyzed for TOC, FAME glucose and ammonium. An example of Micro24 small scale fermentation data from the classically improved strains is shown in **Table 6.** The twice mutagenized and cytostat-selected strain NH-5783 had significantly increased DHA as a percent of fatty acids as well as DHA productivity with respect to its progenitor strain WH-5554. UV treated derivatives of NH-5783 (NH-6161 and NH-61681) demonstrated further significant increases in both DHA as a percent of FAME and DHA production rate.

**Table 6. DHA productivity of classically improved strains in small scale fermentation.**

| **Strain** | **WH-5554** | **NH-5783** | **NH-6161** | **NH-6181** |
|---|---|---|---|---|
| **DHA (mg/l/h)** | 133±2.6 | 159.3.2±2.9 | 199.2±21.4 | 212.9±3.3 |
| **DHA/FAME (%)** | 24.6±0.5 | 32.6±0.5 | 39.2±0.7 | 39.5±0.5 |

### Example 8. Phylogeny of isolated strains.

Three genetic loci, 18SrDNA, actin, and β-tubulin, were studied to establish a phylogenetic tree as per Tsui et al. (Molecular Phylogenetics and Evolution 50: 129-140 (2007)). All thraustochytrid reference genera were included in the analysis with the exception of *Biocosoeca* sp. and *Caecitellus* sp. For each locus, four methods of tree construction were performed: maximum likelihood, maximum parsimony, minimum evolution, and neighbor joining. For convenience, only the most rigorous method (maximum likelihood) is provided for the 18S rDNA sequences, in **Figure 8.** Pairwise distance results demonstrate a close relationship between strains WH-5554 and WH-5628. The closest relative of these strains appears to be *Aurantiochytrium mangrovei* (basionym: *Schizochytrium mangrovei*). *Schizochytrium* sp. ATCC 20888 is also closely related to strains WH-5554 and WH-5628, although not as closely related as *Aurantiochytrium mangrovei.* Based on the barcoding gap differential for the three genetic loci, the new isolates designated herein as WH-5554 and WH-5628 and deposited with the ARS culture collection under NRRL-50834 (strain WH-5554) and NRRL-50835 (strain WH-5628), are proposed to be *Aurantiochytrium* species. Pairwise distance results demonstrate a close relationship between strains WH-5554 and WH-5628.
Lipid profiles of the newly isolated strains confirm this. Yokoyama and Honda (Mycoscience 48: 199-211 (2007)) define *Aurantiochytrium* species as having 5% or less of FAME lipids as arachidonic acid (ARA), and up to about 80% of FAME lipids as DHA. In contrast, *Schizochytrium* species have an ARA content of about 20% FAME. **Table 8** provides the results of analysis of the fatty acid composition of crude microbial oil isolated from NH-6161.

**Table 8. Fatty Acids (%FAME) in crude microbial oil**

| | Average % of Fatty Acids |
|---|---|
| DHAn3 C22:6 n3 | 38.39% |
| DPAn6 C22:5 n6 | 9.21% |
| myristic C14:0 | 25.36% |
| palmitic C16:0 | 19.51% |
| EPA C20:5 n3 | 0.60% |
| ARA C20:4 n6 | 0.16% |

In addition, analysis of the carotenoids of isolated strain WH-5628 and strain NH-5783, derived from isolated strain WH-5554 (see Example 6), demonstrated that both strains produce the carotenoids echinenone, canthaxanthin, phoenicoxanthin, and astaxanthin (**Table 9** and **Figure 9**), which are characteristic of *Aurantiochytrium* species but lacking in *Schizochytrium* species (Yokoyama and Honda (2007)).

**Table 9. Carotenoid content of Labyrinthulomycete Isolates**

| Strain | Tocotrienol, alpha | b-Carotene | Echinenone | Canthaxanthin | Astaxanthin 20:4 | Astaxanthin (16:0/16:0) |
|---|---|---|---|---|---|---|
| WH-5628 (886) | | + | + | + | + | + |
| NH-5783 (derivative of WH-5554) | | + | | + | | + |

Finally, cells of the isolated strain WH-5628 and WH-5554-derived strain NH-5783 were observed microscopically during vegetative growth (**Figure 10**). Consistent with the morphological description of *Aurantiochytrium* by Yokoyama and Honda (2007), vegetative cells of WH-5628 and NH-5783 were found to be dispersed as single cells and were not found in the large aggregates characteristic of the *Schizochytrium.* Cultures propagated in liquid medium at 15°C were visibly pigmented after propagation for 60 hours, a phenotype consistent with identification of both NH-5783 (and by extension, parental strain WH-5554) and WH-5628 as *Aurantiochytrium* and not *Schizochytrium.*

### SEQUENCES

SEQ ID NO:1
   DNA
   *Aurantiochytrium sp.*
   18S rDNA Fragment 1 from strain WH-05554
SEQ ID NO:2
   DNA
   *Aurantiochytrium sp.*
   18S rDNA Fragment 2 from strain WH-05554
SEQ ID NO:3
   DNA
   *Aurantiochytrium sp.*
   18S rDNA Fragment 3 from strain WH-05554
SEQ ID NO:4
   DNA
   *Aurantiochytrium sp.*
   18S rDNA Fragment 4 from strain WH-05554
SEQ ID NO:5
   DNA
   *Aurantiochytrium sp.*
   18S rDNA Fragment 1 from strain NH-05783
SEQ ID NO:6
   DNA
   *Aurantiochytrium sp.*
   18S rDNA Fragment 2 from strain NH-05783
SEQ ID NO:7
   DNA
   *Aurantiochytrium sp.*
   18S rDNA Fragment 3 from strain NH-05783
SEQ ID NO:8
   DNA
   *Aurantiochytrium sp.*
   18S rDNA Fragment 4 from strain NH-05783
SEQ ID NO:9
   DNA
   *Aurantiochytrium sp.*
   18S rDNA Fragment 1 from strain NH-06161
SEQ ID NO:10
   DNA
   *Aurantiochytrium sp.*
   18S rDNA Fragment 2 from strain NH-06161
SEQ ID NO:11
   DNA
   *Aurantiochytrium sp.*
   18S rDNA Fragment 3 from strain NH-06161
SEQ ID NO:12
   DNA
   *Aurantiochytrium sp.*
   18S rDNA Fragment 4 from strain NH-06161

## Claims

1. A microbial oil produced by a mutant labyrinthulomycete microrganism, wherein the labyrinthulomycete microorganism is a strain of Thraustochytrium, Schizochytrium, or Aurantiochytrium, wherein the microbial oil comprises at least 35% of the total fatty acids as docosahexaenoic acid (DHA) and at least 25% of the total fatty acids as myristic acid, and wherein the microorganism has been subjected to UV treatment.

2. A microbial oil according to claim 1, wherein the ratio of DHA to docosapentaenoic acid (DPA) is at least 3.5 : 1.

3. A microbial oil according to claim 1, wherein: 10% or less of the total fatty acids comprise docosapentaenoic acid (DPA); wherein the total fatty acids comprise less than 2% arachidonic acid (ARA); and/or wherein the total fatty acids comprise less than 1% eicosapentaenoic acid (EPA).

4. A microbial oil according to claim 1, wherein the microbial oil is a crude oil or a refined oil.

5. A product comprising a microbial oil according to claim 1.

6. A product according to claim 5, wherein the product is a food product, an animal feed product, a cosmetic product, or a pharmaceutical product.

7. A mutant *Thraustochytrium, Schizochytrium,* or *Aurantiochytrium* microorganism that produces a microbial oil according to claim 1, wherein the microorganism has been subjected to UV treatment.

8. A mutant Thraustochytrium, Schizochytrium, or Aurantiochytrium microorganism according to claim 7, wherein the mutant microorganism produces DHA in a small scale culture at a rate of at least 150 mg/L/h.

9. A mutant Thraustochytrium, Schizochytrium, or Aurantiochytrium microorganism according to claim 8, wherein the mutant microorganism produces at least 20% more DHA and at least 100% more myristic acid as a percent of total fatty acids than the wild type strain from which it is derived.

10. A mutant Thraustochytrium, Schizochytrium, or Aurantiochytrium microorganism according to claim 7, comprising an 18S rDNA sequence having at least 98% identity to a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, and SEQ ID NO:12.

11. A mutant Thraustochytrium, Schizochytrium, or Aurantiochytrium microorganism according to claim 10, wherein the mutant microorganism comprises an 18S rDNA sequence having at least 98% identity to:
a sequence selected from SEQ ID NO:5 and SEQ ID NO:9;
a sequence selected from SEQ ID NO:6 and SEQ ID NO:10;
a sequence selected from SEQ ID NO:7 and SEQ ID NO:11; or
a sequence selected from SEQ ID NO:8 and SEQ ID NO:12.

12. A mutant Thraustochytrium, Schizochytrium, or Aurantiochytrium microorganism according to claim 11, wherein the mutant microorganism comprises an 18S rDNA sequence having at least 98.5% identity to:
a sequence selected from SEQ ID NO:5 and SEQ ID NO:9;
a sequence selected from SEQ ID NO:6 and SEQ ID NO:10;
a sequence selected from SEQ ID NO:7 and SEQ ID NO:11; or
a sequence selected from SEQ ID NO:8 and SEQ ID NO:12.

13. A microbial biomass comprising the mutant microorganism of claim 7.

14. A product comprising a microbial biomass according to claim 13.

## Patentansprüche

1. Mikrobielles Öl, durch einen mutierten Netzschleimpilz-Mikroorganismus hergestellt, wobei der Netzschleimpilz-Mikroorganismus ein Stamm von Thraustochytrium, Schizochytrium oder Aurantiochytrium ist, wobei das mikrobielle Öl zu mindestens 35 % der gesamten Fettsäuren Docosahexaensäure (DHA) und zu mindestens 25 % der gesamten Fettsäuren Myristinsäure umfasst, und wobei der Mikroorganismus einer UV-Behandlung ausgesetzt worden ist.

2. Mikrobielles Öl nach Anspruch 1, wobei das Verhältnis von DHA zu Docosapentaensäure (DPA) mindestens 3,5:1 beträgt.

3. Mikrobielles Öl nach Anspruch 1, wobei:
10 % oder weniger der Gesamtfettsäuren Docosapentaensäure (DPA) umfassen;
wobei die Gesamtfettsäuren weniger als 2 % Arachidonsäure (ARA) umfassen; und/oder
wobei die Gesamtfettsäuren weniger als 1 % Eicosapentaensäure (EPA) umfassen.

4. Mikrobielles Öl nach Anspruch 1, wobei das mikrobielle Öl ein Rohöl oder ein raffiniertes Öl ist.

5. Erzeugnis, das ein mikrobielles Öl nach Anspruch 1 umfasst.

6. Erzeugnis nach Anspruch 5, wobei das Erzeugnis ein Lebensmittelerzeugnis, ein Tierfuttererzeugnis, ein kosmetisches Erzeugnis oder ein pharmazeutisches Erzeugnis ist.

7. Mutierter *Thraustochytrium-, Schizochytrium-* oder *Aurantiochytrium-Mikroorganismus,* der ein mikrobielles Öl nach Anspruch 1 erzeugt, wobei der Mikroorganismus einer UV-Behandlung unterzogen worden ist.

8. Mutierter Thraustochytrium-, Schizochytrium- oder Aurantiochytrium-Mikroorganismus nach Anspruch 7, wobei der mutierte Mikroorganismus DHA in einer Kultur im kleinen Maßstab mit einer Geschwindigkeit von mindestens 150 mg/l/h erzeugt.

9. Mutierter Thraustochytrium-, Schizochytrium- oder Aurantiochytrium-Mikroorganismus nach Anspruch 8, wobei der mutierte Mikroorganismus mindestens 20 % mehr DHA und mindestens 100 % mehr Myristinsäure erzeugt als der Wildtyp-Stamm, von dem er stammt.

10. Mutierter Thraustochytrium-, Schizochytrium- oder Aurantiochytrium-Mikroorganismus nach Anspruch 7, der eine 18S-rDNA-Sequenz mit mindestens 98 % Identität zu einer Sequenz umfasst, ausgewählt aus der Gruppe bestehend aus SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11 und SEQ ID NO:12.

11. Mutierter Thraustochytrium-, Schizochytrium- oder Aurantiochytrium-Mikroorganismus nach Anspruch 10, wobei der mutierte Mikroorganismus eine 18S-rDNA-Sequenz mit mindestens 98 % Identität zu Folgendem aufweist:
einer Sequenz, ausgewählt aus SEQ ID NO:5 und SEQ ID NO:9;
einer Sequenz, ausgewählt aus SEQ ID NO:6 und SEQ ID NO:10;
einer Sequenz, ausgewählt aus SEQ ID NO:7 und SEQ ID NO:11; oder
einer Sequenz, ausgewählt aus SEQ ID NO:8 und SEQ ID NO:12.

12. Mutierter Thraustochytrium-, Schizochytrium- oder Aurantiochytrium-Mikroorganismus nach Anspruch 11, wobei der mutierte Mikroorganismus eine 18S-rDNA-Sequenz mit mindestens 98,5 % Identität zu Folgendem aufweist:
einer Sequenz, ausgewählt aus SEQ ID NO:5 und SEQ ID NO:9;
einer Sequenz, ausgewählt aus SEQ ID NO:6 und SEQ ID NO:10;
einer Sequenz, ausgewählt aus SEQ ID NO:7 und SEQ ID NO:11; oder
einer Sequenz, ausgewählt aus SEQ ID NO:8 und SEQ ID NO:12.

13. Mikrobielle Biomasse, die den mutierten Mikroorganismus nach Anspruch 7 umfasst.

14. Erzeugnis, das eine mikrobielle Biomasse nach Anspruch 13 umfasst.

## Revendications

1. Huile microbienne produite par un micro-organisme labyrinthulomycète mutant, le micro-organisme labyrinthulomycète étant une souche de Thraustochytrium, de Schizochytrium ou d'Aurantiochytrium, l'huile microbienne comprenant au moins 35 % des acides gras totaux sous forme d'cide docosahexaénoïque (DHA) et au moins 25 % des acides gras totaux sous forme d'acide myristique et le micro-organisme ayant été soumis à un traitement aux UV.

2. Huile microbienne selon la revendication 1, dans laquelle le rapport entre le DHA et l'acide docosapentaénoïque (DPA) est d'au moins 3,5:1.

3. Huile microbienne selon la revendication 1, dans laquelle :
10 % au plus des acides gras totaux comprennent l'acide docosapentaénoïque (DPA) ; dans laquelle le total des acides gras comprend moins de 2 % d'acide arachidonique (ARA) ; et/ou
dans laquelle le total des acides gras comprend moins de 1 % d'acide eicosapentaénoïque (EPA).

4. Huile microbienne selon la revendication 1, l'huile microbienne étant une huile brute ou une huile raffinée.

5. Produit comprenant une huile microbienne selon la revendication 1.

6. Produit selon la revendication 5, dans lequel le produit est un produit alimentaire, un aliment pour animaux, un produit cosmétique ou un produit pharmaceutique.

7. Micro-organisme mutant de *Thraustochytrium, de Schizochytrium* ou d'*Aurantiochytrium* qui produit une huile microbienne selon la revendication 1, le micro-organisme ayant été soumis à un traitement aux UV.

8. Micro-organisme mutant de Thraustochytrium, de Schizochytrium ou d'Aurantiochytrium selon la revendication 7, le micro-organisme mutant produisant du DHA dans une culture à petite échelle à un taux d'au moins 150 mg/L/h.

9. Micro-organisme mutant de Thraustochytrium, de Schizochytrium ou d'Aurantiochytrium selon la revendication 8, le micro-organisme mutant produisant au moins 20 % en plus de DHA et au moins 100 % en plus d'acide myristique, en pourcentage du total d'acides gras totaux, par rapport à la souche de type sauvage dont il est dérivé.

10. Micro-organisme mutant de Thraustochytrium, de Schizochytrium ou d'Aurantiochytrium selon la revendication 7, comprenant une séquence d'ADNr 18S ayant au moins 98 % d'identité avec une séquence choisie dans le groupe constitué par SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11 et SEQ ID NO:12.

11. Micro-organisme mutant de Thraustochytrium, de Schizochytrium ou d'Aurantiochytrium selon la revendication 10, dans lequel le micro-organisme mutant comprend une séquence d'ADNr 18S ayant au moins 98 % d'identité avec :
une séquence choisie parmi SEQ ID NO:5 et SEQ ID NO:9 ;
une séquence choisie parmi SEQ ID NO:6 et SEQ ID NO:10 ;
une séquence choisie parmi SEQ ID NO:7 et SEQ ID NO:11 ; ou
une séquence choisie parmi SEQ ID NO:8 et SEQ ID NO:12.

12. Micro-organisme mutant de Thraustochytrium, de Schizochytrium ou d'Aurantiochytrium selon la revendication 11, dans lequel le micro-organisme mutant comprend une séquence d'ADNr 18S ayant au moins 98,5 % d'identité avec :
une séquence choisie parmi SEQ ID NO:5 et SEQ ID NO:9 ;
une séquence choisie parmi SEQ ID NO:6 et SEQ ID NO:10 ;
une séquence choisie parmi SEQ ID NO:7 et SEQ ID NO:11 ; ou
une séquence choisie parmi SEQ ID NO:8 et SEQ ID NO:12.

13. Biomasse microbienne comprenant le micro-organisme mutant selon la revendication 7.

14. Produit comprenant une biomasse microbienne selon la revendication 13.
